# EUROPEAN PATENT APPLICATION

(11) **EP 1 400 533 A1**
(43) Date of publication of application: **24.03.2004**
(21) Application number: 02020425.1
(22) Date of filing: 11.09.2002
(51) Int. Cl.: C07K 14/505, C07K 17/10, C08B 31/00, C07K 17/06, C12N 15/12, A61K 38/18, C07K 14/55, C07K 14/54, C07K 14/56, C07K 14/565, C07K 14/53

(54) **HASylated polypeptides, especially HASylated erythropoietin**

(71) Applicant: Fresenius Kabi Deutschland GmbH, 61346 Bad Homburg v.d.H. (DE)
(72) Inventor: Conradt, Harald S., 38100 Braunschweig (DE); Grabenhorst, Eckart, 38104 Braunschweig (DE); Nimtz, Manfred, 38300 Wolfenbüttel (DE); Zander, Norbert, 38524 Sassenburg (DE); Frank, Ronald, 38527 Meine-Grassel (DE); Eichner, Wolfram, 35510 Butzbach (DE)
(74) Representative: Wichmann, Hendrik, Dr.

(57) **Abstract**

The present invention relates to hydroxyalkylstarch (HAS)-polypeptide-conjugate (HAS-polypeptide) comprising one or more HAS moecules, wherein each HAS is conjugated to the polypeptide via a carbohydrate moiety or a thioether as well as to methods for the production thereof. In a preferred embodiment, the polypeptide is erythropoietin (EPO).

## Description

The present invention relates to polypeptides, especially erythropoietin conjugated to hydroxyalkylstarch (HAS), especially to hydroxyethylstarch.

The application of polypeptides, especially enzymes or cytokines, to the circulatory system in order to obtain a particular physiological effect is a well-known tool in modem medicine.

Erythropoietin (EPO) is a glycoprotein hormone necessary for the maturation of erythroid progenitor cells into erythrocytes. In human adults, it is produced in the kidney. EPO is essential in regulating the level of red blood cells in the circulation. Conditions marked by low levels of tissue oxygen provoke an increased biosynthesis of EPO, which in turn stimulates erythropoiesis. A loss of kidney function as it is seen in chronic renal failure, for example, typically results in decreased biosynthesis of EPO and a concomitant reduction in red blood cells.

Erythropoietin is an acid glycoprotein hormone of approximately 34,000 Da. Human erythropoietin is a 166 amino acid polypeptide that exists naturally as a monomer (Lin et al., 1985, PNAS 82, 7580-7584, EP 148 605 B2, EP 411 678 B2). The identification, cloning and expression of genes encoding erythropoietin are described, e.g., in U.S. Patent 4,703,008. The purification of recombinant erythropoietin from cell culture medium that supported the growth of mammalian cells containing recombinant erythropoietin plasmids, for example, is described in U.S. Patent 4,667,016.

It is generally believed in this technical field that the biological activity of EPO in vivo mainly depends on the degree of sialic acids bound to EPO (see e.g. EP 428 267 B1). Theoretically, 14 molecules of sialic acid can be bound to one molecule EPO at the terminal ends of the carbohydrate side chains linked to N- and O-glycosylation sites. Highly sophisticated purification steps are necessary to obtain highly sialylated EPO preparations.

For further detailed information on erythropoietin see Krantz, Erythropoietin, 1991, Blood, 77(3):419-34 (Review) and Cerami, Beyond erythropoiesis: novel applications for recombinant human erythropoietin, 2001, Semin Hematol., (3 Suppl 7):33-9 (Review).

A well-known problem with the application of polypeptides and enzymes is that these proteins often exhibit an unsatisfactory stability. Especially, erythropoietin has a relatively short plasma half live (Spivak and Hogans, 1989, Blood 73, 90; McMahon et al., 1990, Blood 76, 1718). This means that therapeutic plasma levels are rapidly lost and repeated intravenous administrations must be carried out. Furthermore, in certain circumstances an immune response against the peptides is observed.

It is generally accepted that the stability of polypeptides can be improved and the immune response against these polypeptides is reduced when the polypeptides are coupled to polymeric molecules. WO 94/28024 discloses that physiologically active polypeptides modified with polyethyleneglycol (PEG) exhibit reduced immunogenicity and antigenicity and circulate in the bloodstream considerably longer than unconjugated proteins, i.e. have a longer clearance rate.

However, PEG-drug conjugates exhibit several disadvantages, e.g. they do not exhibit a natural structure which can be recognized by elements of in vivo degradation pathways. Therefore, apart from PEG-conjugates, other conjugates and protein polymerates have been produced. A plurality of methods for the cross-linking of different proteins and macromolecules such as polymerase have been described in the literature (see e.g. Wong, Chemistry of protein conjugation and cross-linking, 1993, CRCS, Inc.).

Hydroxyethylstarch (HES) is a derivative of naturally occurring amylopektine and is degraded by α-Amylase in the body. The preparation of HES-protein-conjugates is described in the state of the art (see, e.g., HES-hemoglobin-conjugates in DE 26 16 086 or DE 26 46 854).

DE 26 46 854 discloses methods for the conjugation of hemoglobin to HES. In these methods, HES is reacted with sodiumperiodate, which results in the production of dialdehydes which are linked to hemoglobin. In contrast to this, DE 26 16 086 discloses the conjugation of hemoglobin to HES according to a procedure wherein first a cross-linking agent (e.g. bromocyane) is bound to HES and subsequently hemoglobin is linked to the intermediate product.

HES is a substituted derivative of the carbohydrate polymer amylopektine, which is present in corn starch at a concentration of up to 95 % per weight. HES exhibits advantageous biological properties and is used as a blood volume replacement agent and in hemodilution therapy in the clinics (Sommermeyer et al., 1987, Krankenhauspharmazie, 8(8), 271-278; and Weidler et al., 1991, Arzneim.-Forschung/Drug Res., 41, 494-498).

Amylopektine consists of glucose moieties, wherein in the main chain α-1,4-glycosidic bonds are present and at the branching sites α-1,6-glycosidic bonds are found. The physical-chemical properties of this molecule are mainly determined by the type of glycosidic bonds. Due to the nicked α-1,4-glycosidic bond, helical structures with about six glucose-monomers per turn are produced.

The physical-chemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

Consequently, HES is mainly characterized by the molecular weight distribution and the degree of substitution. There are two possibilities of describing the substitution degree:
1. The substitution degree can be described relative to the portion of substituted glucose monomers with respect to all glucose moieties (DS).
2. The substitution degree can be described as the "molar substitution" (MS), wherein the number of hydroxyethyl groups per glucose moiety are described.

HES solutions are present as polydisperse compositions, wherein each molecule differs from the other with respect to the polymerisation degree, the number and pattern of branching sites and the substitution pattern. HES is therefore a mixture of compounds with different molecular weight. Consequently, a particular HES solution is determined by average molecular weight with the help of statistical means. In this context, Mₙ is calculated as the arithmetic mean depending on the number of molecules. Alternatively, M_{w}, the weight mean, represents a unit which depends on the mass of the HES.

The HES-drug conjugates disclosed in the art suffer from the disadvantage that HES is not conjugated site-specifically to the drug. Consequently, the conjugation results in a very heterogenous product having many components that may be inactive due to the destruction of the 3-dimensional structure during the conjugation step.

In summary, there is still a need for further improved polypeptides with improved stability and/or bioactivity. This applies especially to erythropoietin where isoforms with a high degree of sialic acids and therefore high actvity have to be purified from isoforms with a low degree of sialic acids (see EP 428 267 B1). Therefore, it would be highly advantageous if production methods were available which provide highly active polypeptides without requiring extensive purification. Unfortunately, the production of polypeptides in bacteria or insect cells is often difficult, because the polypeptides are often not produced in a properly folded, native confirmation and lack proper glycosylation.

Consequently, it is an object of the present invention to provide polypeptide derivatives, especially erythropoietin derivatives, having a high biological activity in vivo which can be easily produced and at reduced costs. Furthermore, it is a further object of the present invention to provide a method for the production of polypeptide derivatives which is easy to perform and yields in products with high biological activity. It is a further object of the invention to provide pharmaceutical compositions comprising polypeptide derivatives with high biological activity.

According to one aspect of the present invention, the problem is solved by a hydroxyalkylstarch (HAS)-erythropoietin (EPO)-conjugate (HAS-EPO) comprising one or more HAS molecules, wherein each HAS is conjugated to the EPO via
a) a carbohydrate moiety; or
b) a thioether.

The HAS-EPO of the invention has the advantage that it exhibits an improved biological stability when compared to the erythropoietin before conjugation. This is mainly due to the fact that HAS-EPO is less or even not recognized by the removal systems of the liver and kidney and therefore persists in the circulatory system for a longer period of time. Furthermore, since the HAS is attached site-specifically, the risk of destroying the in vivo biological activity of EPO by conjugation of HAS to EPO is minimized.

The HAS-EPO of the invention has mainly two components, namely the erythropoietin (EPO)-polypeptide and the hydroxyalkylstarch (HAS) linked thereto.

The EPO can be of any human (see e.g. Inoue, Wada, Takeuchi, 1994, An improved method for the purification of human erythropoietin with high in vivo activity from the urine of anemic patients, Biol Pharm Bull. 17(2), 180-4; Miyake, Kung, Goldwasser, 1977, Purification of human erythropoietin., J Biol Chem., 252(15), 5558-64) or another mammalian source and can be obtained by purification from naturally occurring sources like human kidney, embryonic human liver or animal, preferably monkey kidney. Furthermore, the expression "erythropoietin" or "EPO" encompasses also an EPO variant wherein one or more amino acids (e.g. 1 to 25, preferably 1 to 10, more preferred 1 to 5, most preferred 1 or 2) have been exchanged by another amino acid and which exhibits erythropoietic activity (see e.g. EP 640 619 B1). The measurement of erythropoietic activity is described in the art (for measurement of activity in vitro see e.g. Fibi et al.,1991, Blood, 77, 1203 ff; Kitamura et al, 1989, J. Cell Phys., 140, 323-334; for measurement of EPO activity in vivo see Ph. Eur. 2001, 911-917; Ph. Eur. 2000, 1316 Erythropoietini solutio concentrata, 780- 785; European Pharmacopoeia (1996/2000); European Pharmacopoeia, 1996, Erythropoietin concentrated solution, Pharmaeuropa., 8, 371-377; Fibi, Hermentin, Pauly, Lauffer, Zettlmeissl., 1995, N- and O-glycosylation muteins of recombinant human erythropoietin secreted from BHK-21 cells, Blood, 85(5), 1229-36; (EPO and modified EPO forms were injected into female NMRI mice (equal amounts of protein 50 ng/mouse) at day 1, 2 and 3 blood samples were taken at day 4 and reticulocytes were determined)). Further publications where tests for the measurement of the activity of EPO are described Barbone, Aparicio, Anderson, Natarajan, Ritchie, 1994, Reticulocytes measurements as a bioassay for erythropoietin, J. Pharm. Biomed. Anal., 12(4), 515-22; Bowen, Culligan, Beguin, Kendall, Villis, 1994, Estimation of effective and total erythropoiesis in myelodysplasia using serum transferrin receptor and erythropoietin concentrations, with automated reticulocyte parameters, Leukemi, 8(1), 151-5; Delorme, Lorenzini, Giffin, Martin, Jacobsen, Boone, Elliott, 1992, Role o glycosylation on the secretion and biological activity of erythropoietin, Biochemistry, 31(41), 9871-6; Higuchi, Oh-eda, Kuboniwa, Tomonoh, Shimonaka, Ochi, 1992;Role of sugar chains in the expression of the biological activity of human erythropoietin, J. Biol. Chem., 267(11), 7703-9; Yamaguchi, Akai, Kawanishi, Ueda, Masuda, Sasaki, 1991, ffects of site-directed removal of N-glycosylation sites in human erythropoietin on its production and biological properties, J. Biol. Chem., 266(30), 20434-9; Takeuchi, Inoue, Strickland, Kubota, Wada, Shimizu, Hoshi, Kozutsumi, Takasaki, Kobata, 1989, Relationship between sugar chain structure and biological activity of recombinant human erythropoietin produced in Chinese hamster ovary cells, Proc. Natl. Acad. Sci. USA, 85(20), 7819-22; Kurtz, Eckardt, 1989, Assay methods for erythropoietin, Nephron., 51(1), 11-4 (German); Zucali, Sulkowski, 1985, Purification of human urinary erythropoietin on controlled-pore glass and silicic acid, Exp. Hematol., 13(3), 833-7; Krystal, 1983, Physical and biological characterization of erythroblast enhancing factor (EEF), a late acting erythropoetic stimulator in serum distinct from erythropoietin, Exp. Hematol., 11(1), 18-31.

Preferably, the EPO is recombinantly produced. This includes the production in eukaryotic or prokaryotic cells, preferably mammalian, insect, yeast, bacterial cells or in any other cell type which is convenient for the recombinant production of EPO. Furthermore, the EPO may be expressed in transgenic animals (e.g. in body fluids like milk, blood, etc.), in eggs of transgenic birds, especially poultry, preferred chicken, or in transgenic plants.

The recombinant production of a polypeptide is known in the art. In general, this includes the transfection of host cells with an appropriate expression vector, the cultivation of the host cells under conditions which enable the production of the polypeptide and the purification of the polypeptide from the host cells. For detailled information see e.g. Krystal, Pankratz, Farber, Smart, 1986, Purification of human erythropoietin to homogeneity by a rapid five-step procedure, Blood, 67(1), 71-9; Quelle, Caslake, Burkert, Wojchowski, 1989, High-level expression and purification of a recombinant human erythropoietin produced using a baculovirus vector, Blood, 74(2), 652-7; EP 640 619 B1 and EP 668 351 B1.

In a preferred embodiment, the EPO has the amino acid sequence of human EPO (see EP 148 605 B2).

The EPO may comprise one or more carbohydrate side chains (preferably 1-4, preferably 4) attached to the EPO via N- and/ or O-linked glycosylation, i.e. the EPO is glycosylated. Usually, when EPO is produced in eukaryotic cells, the polypeptide is posttranslationally glycosylated. Consequently, the carbohydrate side chains may have been attached to the EPO during biosynthesis in mammalian, especially human, insect or yeast cells. The structure and properties of glycosylated EPO have been extensively studied in the art (see EP 428 267 B1; EP 640 619 B1; Rush, Derby, Smith, Merry, Rogers, Rohde, Katta, 1995, Microheterogeneity of erythropoietin carbohydrate structure, Anal Chem., 67(8), 1442-52; Takeuchi, Kobata, 1991, Structures and functional roles of the sugar chains of human erythropoietins, Glycobiology, 1(4), 337-46 (Review).

The HAS may be directly conjugated to the EPO or, alternatively, via a linker molecule. The nature of the linker molecule depends on the way how the HAS is linked to the EPO. Possible functional groups of linkers are described in Table 1 and below. Several linkers are commercially available (e.g. from Pierce, available from Perbio Science Deutschland GmbH, Bonn, Germany)). Some suitable linkers are described in Table 2. The nature of the linker and its purpose are described in detail below in the section concerning the method for the production of HES-EPO.

According to a preferred embodiment of the HAS-EPO conjugate of the invention, the HAS is conjugated to the EPO via a carbohydrate moiety.

In the context of the present invention, the term "carbohydrate moiety" refers to hydroxyaldehydes or hydroxyketones as well as to chemical modifications thereof (see Römpp Chemielexikon, Thieme Verlag Stuttgart, Germany, 9^{th} edition 1990, Volume 9, pages 2281-2285 and the literature cited therein). Furthermore, it also refers to derivatives of naturally occuring carbohydrate moieties like glucose, galactose, mannose, sialic acid and the like. The term also includes chemically oxidized naturally occuring carbohydrate moieties wherein the ring structure has been opened.

The carbohydrate moiety may be linked directly to the EPO polypeptide backbone. Preferably, the carbohydrate moiety is part of a carbohydrate side chain. In this case, further carbohydrate moieties may be present between the carbohydrate moiety to which HAS is linked and the EPO polypeptide backbone. More preferably, the carbohydrate moiety is the terminal moiety of the carbohydrate side chain.

In a more preferred embodiment, the HAS is conjugated to a galactose residue of the carbohydrate side chains, preferably the terminal galactose residue of the carbohydrate side chain. This galactose residue can be made available for conjugation by removal of terminal sialic acids, followed by oxidation (see below).

In a further more preferred embodiment, the HAS is conjugated to a sialic acid residue of the carbohydrate side chains, preferably the terminal sialic acid residue of the carbohydrate side chain.

Furthermore, the HAS may be conjugated to the EPO via a thioether. As explained in detail below, the S atom can be derived from any SH group attached to the EPO, both naturally or non naturally occurring.

In a preferred embodiment, the S atom may be derived from a SH group which has been introduced in an oxidized carbohydrate moiety of HES, preferably an oxidized carbohydrate moiety which is part of a carbohydrate side chain of EPO (see below).

Preferably, the S atom in the thioether is derived from a naturally-occurring cysteine or from an added cysteine. More preferably, the EPO has the amino acid sequence of human EPO and the naturally occurring cysteines are cysteine 29 and/ or 33. In a more preferred embodiment, HAS is conjugated to cysteine 29 and cysteine 33 is replaced by another amino acid. Alternatively, HAS may be conjugated to cysteine 33 and cysteine 29 is replaced by another amino acid.

In the context of the present invention, by the term "added cysteines" it is meant that the polypeptides, preferably EPO, comprise a cysteine residue which is not present in the wild-type polypeptide.

In the context of this aspect of the invention, the cysteine may be an additional amino acid added at the N- or C-terminal end of EPO.

Furthermore, the added cysteine may have been added by replacing a naturally occuring amino acid by a cysteine. Suitable methods are known in the art (see above). Preferably, in the context of this aspect of the invention, the EPO is human EPO and the replaced amino acid residue is serine 126.

The second component of the HAS-EPO is hydroxyalkylstarch (HAS).

In the context of the present invention, the term "hydroxyalkylstarch" is used to indicate starch derivatives which have been substituted by hydroxyalkylgroups. In this context, the alkyl group may be substituted. Preferably, the hydroxyalkyl contains 2-10 carbon atoms, more preferably 2-4 carbon atoms. "Hydroxyalkylstarch" therefore preferably comprises hydroxyethylstarch, hydroxypropylstarch and hydroxybutylstarch, wherein hydroxyethylstarch and hydroxypropylstarch are preferred.

The hydroxyalkylgroup(s) of HAS contain at least one OH-group.

The expression "hydroxyalkylstarch" also includes derivatives wherein the alkyl group is mono- or polysubstituted. In this context, it is preferred that the alkyl group is substituted with an halogen, especially flourine, or with an aryl group, provided that the HAS remains water soluble. Furthermore, the terminal hydroxy group of hydroxyalkyl may be esterified or etherified. In addition, the alkyl group of the hydroxyalkylstarch may be linear or branched.

Furthermore, instead of alkyl, also linear or branched substituted or unsubstituted alkene groups may be used.

Hydroxyethylstarch (HES) is most preferred for all embodiments of the present invention.

In the context of the present invention, hydroxyethylstarch may have a mean molecular weight (weight mean) of 1-300 kDa, wherein a mean molecular weight of 5-100 kDa is more preferred. Hydroxyethylstarch can further exhibit a molar degree of substitution of 0.1 to 0.8 and a ratio between C₂:C₆-substitution in the range of 2-20, with respect to the hydroxyethylgroups.

The HAS-EPO may comprise 1-12, preferably 1-9, 1-6 or 1-3, most preferred 1-4 HAS molecules per EPO molecule. The number of HAS-molecules per EPO molecule can be determined by quanatitative carbohydrate compositional analysis using GC-MS after hydrolysis of the product and derivatisation of the resulting monosaccharides (see Chaplin and Kennedy (eds.), 1986, Carbohydrate Analysis: a practical approach, IRL Press Practical approach series (ISBN 0-947946-44-3), especially Chapter 1, Monosaccharides, page 1-36; Chapter 2, Oligosaccharides, page 37-53, Chapter 3, Neutral Polysaccharides, page 55-96).

The HAS-EPO conjugate of the invention may exhibit essentially the same in-vitro biological activity as recombinant native EPO, since the in-vitro biological activity only measures binding affinity to the EPO receptor. Methods for determining the in-vitro biological activity are known in the art (see above).

Furthermore, the HAS-EPO exibits a greater in vivo activity than the EPO used as a starting material for conjugation (unconjugated EPO). Methods for determining the in vivo biological activity are known in the art (see above). Furthermore, assays for the determination of in vivo and in vitro EPO activity are given in Examples 9 and 10.

The HAS-EPO conjugate may exhibit an in vivo activity of 110 % to 300 %, preferably 110 % to 200 %, more preferred 110 % to 180 % or 110 to 150 %, most preferred 110 % to 140 %, if the in vivo activity of the unconjugated EPO is set as 100 %.

Compared to the highly sialylated EPO of Amgen (see EP 428 267 B1), the HAS-EPO exibits preferably at least 50%, more preferred at least 70 %, even more preferred at least 85 % or at least 95 %, at least 150 %, at least 200 % or at least 300 % of the in vivo activity of the highly sialylated EPO, if the in vivo activity of highly sialylated EPO is set as 100 %. Most preferred, it exhibits at least 95 % of the in vivo activity of the highly sialylated EPO.

The high in vivo biological activity of the HAS-EPO conjugate of the invention mainly results from the fact that the HAS-EPO conjugate remains longer in the circulation than the unconjugated EPO, because it is less recognized by the removal systems of the liver and because renal clearance is reduced due to the higher molecular weight. Methods for the determination of the in vivo half life time of EPO in the circulation are known in the art (Sytkowski, Lunn, Davis, Feldman, Siekman, 1998, Human erythropoietin dimers with markedly enhanced in vivo activity, Proc. Natl. Acad. Sci. USA, 95(3), 1184-8).

Consequently, it is a great advantage of the present invention that a HAS-EPO is provided that may be administered less frequently than the EPO preparations commercially available at present. While standard EPO preparations have to be administered at least all 3 days, the HAS-EPO conjugate of the invention is preferable adminstered twice a week, more preferably once a week.

All embodiments disclosed below with respect of the method of the invention to produce a HAS-EPO concerning properties of EPO or HAS apply also to the HAS-EPO conjugate of the invention.

Hydroxyalkylstarch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially of acetic acid, In this context, acetylstarch, butylstarch or propylstarch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

In a further aspect, the present invention relates to a method for the production of a hydroxyalkylstarch (HAS)-erythropoietin (EPO)-conjugate (HAS-EPO), comprising the steps of:
a) providing EPO being capable of reacting with modified HAS,
b) providing modified HAS being capable of reacting with the EPO of step a), and
c) reacting the EPO of step a) with the HAS of step b), whereby an HAS-EPO is produced comprising one or more HAS molecules, wherein each HAS is conjugated to the EPO via
   i) a carbohydrate moiety; or
   ii) a thioether.

The method of the invention has the advantage that a HAS-EPO conjugate is produced which exhibits a high biological activity. Furthermore, the method of the invention has the advantage that an effective EPO derivative can be produced at reduced costs since the method does not comprise extensive and time consuming purification steps resulting in low final yield, e.g. it is not necessary to purify away undersialylated EPO forms which are known to exhibit low or no in-vivo biological activity.

Accordingly, in the first step of the method of the invention, an EPO is provided which is capable of reacting with modified HAS.

As used in the present invention, the term "providing" has to be interpreted in the way that after the respective step a molecule (in step a) EPO, in step b) HAS) with the desired properties is available.

In the case of step a), this includes the purification of EPO from natural sources as well as the recombinant production in host cells or organisms, and, if necessary, the modification of the EPO so obtained.

With respect to the EPO being the starting material of the present invention, the same applies as for the erythropoietin being part of the HAS-EPO conjugate of the invention. In this context, the preferred embodiments disclosed above apply also for the method of the invention.

Consequently, in a preferred embodiment, the EPO has the amino acid sequence of human EPO.

Preferably, the EPO is recombinantly produced. This includes the production in eukaryotic or prokaryotic cells, preferably mammalian, insect, yeast, bacterial cells or in any other cell type which is convenient for the recombinant production of EPO. Furthermore, the EPO may be expressed in transgenic animals (e.g. in body fluids like milk, blood, etc.), in eggs of transgenic birds, especially poultry, preferred chicken, or in transgenic plants.

The recombinant production of a polypeptide is known in the art. In general, this includes the transfection of host cells with an appropriate expression vector, the cultivation of the host cells under conditions which enable the production of the polypeptide and the purification of the polypeptide from the host cells (Krystal, Pankratz, Farber, Smart, 1986, Purification of human erythropoietin to homogeneity by a rapid five-step procedure, Blood, 67(1), 71-9; Quelle, Caslake, Burkert, Wojchowski, 1989, High-level expression and purification of a recombinant human erythropoietin produced using a baculovirus vector, Blood, 74(2), 652-7; EP 640 619 B1 and EP 668 351 B1).

The EPO may comprise one or more carbohydrate side chains attached to the EPO via N- and/ or O-linked glycosylation, i.e. the EPO is glycosylated. Unsually, when EPO is produced in eukaryotic cells, the polypeptide is posttranslationally glycosylated. Consequently, the carbohydrate side chains may have been attached to the EPO during production in mammalian, especially human, insect or yeast cells, which may be cells of a transgenic animal (see above), either extracted from the animal or still in the animal.

These carbohydrate side chains may have been chemically or enzymatically modified after the expression in the appropriate cells, e.g. by removing or adding one or more carbohydrate moieties (see e.g. Dittmar, Conradt, Hauser, Hofer, Lindenmaier, 1989, Advances in Protein design; Bloecker, Collins, Schmidt, and Schomburg eds., GBF-Monographs, 12, 231-246, VCH Publishers, Weinheim, New York, Cambridge)

It is the object of the method of the invention to provide an HAS-EPO comprising one or more HAS molecules where the HAS is conjugated to the EPO via a carbohydrate moiety (i) or via a thioether (ii). Consequently, the EPO provided in step a) should have the properties that a conjugation via a carbohydrate moiety and/ or via a thioether is possible. Therefore the EPO after step a) may preferably contain either
(1) at least one reactive group linked, either directly or via a linker molecule, to sulfide groups or carbohydrate moieties, which is capable to react with HES or modified HES,
(2) at least one carbohydrate moiety to which modified HAS can be conjugated, and/or
(3) at least one free SH-group.

With respect to possibility (1) above, the EPO of step a) is preferably obtainable by conjugating an appropriate linker molecule to the SH-group(s) or carbohydrate moieties of EPO. An example for such a modified EPO is provided in Example 4, 2.1. It is important to ensure that the addition of the linker molecule does not damage the EPO. However, this is known to the person skilled in the art.

With respect to possibility (2) above, in a preferred embodiment, the modified HAS is conjugated to the EPO via a carbohydrate moiety.

The carbohydrate moiety may be linked directly to the EPO polypeptide backbone. Preferably, the carbohydrate moiety is part of a carbohydrate side chain. In this case, further carbohydrate moieties may be present between the carbohydrate moiety to which HAS is linked and the EPO polypeptide backbone. More preferably, the carbohydrate moiety is the terminal moiety of the carbohydrate side chain.

Consequently, in a preferred embodiment, the modified HAS is attached (via a linker or not, see below) to carbohydrate chains linked to N- and/ or O-glycosylation sites of EPO.

However, it is also included within the present invention that the EPO contains (a) further carbohydrate moiet(y)ies to which the modified HAS is conjugated. Techniques for attaching carbohydrate moieties to polypeptides, either enzymatically or by genetic engineering, followed by expression in appropriate cells, are known in the art (Berger, Greber, Mosbach, 1986, Galactosyltransferase-dependent sialylation of complex and endo-N-acetylglucosaminidase H-treated core N-glycans in vitro, FEBS Lett., 203(1), 64-8; Dittmar, Conradt, Hauser, Hofer, Lindenmaier, 1989, Advances in Protein design; Bloecker, Collins, Schmidt, and Schomburg eds., GBF-Monographs, 12, 231-246, VCH Publishers, Weinheim, New York, Cambridge).

In a preferred embodiment of the method of the invention, the carbohydrate moiety is oxidized in order to be able to react with the modified HAS. This oxidation can be performed either chemically or enzymatically.

Methods for the chemical oxidation of carbohydrate moieties of polypeptides are known in the art and include the treatment with perjodate (Chamow et al., 1992, J. Biol. Chem., 267, 15916-15922).

By chemically oxidizing, it is principally possible to oxidize any carbohydrate moiety, being terminally positioned or not. However, by choosing mild conditions (1 mM periodate, 0 °C in contrast to harsh conditions: 10 mM periodate 1h at room temperature), it is possible to preferably oxidize the terminal carbohydrate moiety, e.g. sialic acid or galactose, of a carbohydrate side chain.

Alternatively, the carbohydrate moiety may be oxidized enzymatically. Enzymes for the oxidation of the individual carbohydrate moieties are known in the art, e.g. in the case of galactose the enzyme is galactose oxidase.

If it is intended to oxidize terminal galactose moieties, it will be eventually necessary to remove terminal sialic acids (partially or completely) if the EPO has been produced in cells capable of attaching sialic acids to carbohydrate chains, e.g. in mammalian cells or in cells which have been genetically modified to be capable of attaching sialic acids to carbohydrate chains. Chemical or enzymatic methods for the removal of sialic acids are known in the art (Chaplin and Kennedy (eds.), 1996, Carbohydrate Analysis: a practical approach, especially Chapter 5 Montreuill, Glycoproteins, pages 175-177; IRL Press Practical approach series (ISBN 0-947946-44-3)).

However, it is also included within the present invention that the carbohydrate moiety to which the modified HAS is to be attached is attached to the EPO within step a). In the case it is desired to attach galactose, this can be achieved by the means of galactosyltransferase. The methods are known in the art (Berger, Greber, Mosbach, 1986, Galactosyltransferase-dependent sialylation of complex and endo-N-acetylglucosaminidase H-treated core N-glycans in vitro, FEBS Lett., 203(1), 64-8).

In a most preferred embodiment, in step a) the EPO is modified by oxidizing at least one terminal saccharide unit, preferably galactose, of the one or more carbohydrate side chains of the EPO, preferably after partial or complete (enzymatic and/or chemical) removal of the terminal sialic acid, if necessary (see above).

Consequently, preferably the modified HAS is conjugated to the oxidized terminal saccharide unit of the carbohydrate chain, preferably galactose.

Furthermore, the modified HAS may be preferably conjugated to a terminal sialic acid, which is preferably oxidized in step a) of the method of the invention.

In a further preferred embodiment (see point (3) above), the EPO comprises at least one free SH-group.

According to a preferred embodiment, this SH group may be linked to a preferably oxidized carbohydrate moiety, e.g. by using a hydroxylamine derivative, e.g. 2-(aminooxy)ethylmercaptan hydrochloride (Bauer L. et al., 1965, J. Org. Chem., 30, 949) or by using a hydrazide derivative, e.g. thioglycolic acid hydrazide (Whitesides et al., 1977, J. Org. Chem., 42, 332.) The methods for conjugating these molecules to the oxidized carbohydrate moiety of EPO may be analogous to those described in Example Protocols 8 and 9.

According to a further preferred embodiment, the free SH-group is part of a naturally-occurring cysteine or of an added cysteine.

Mammalian EPO has several cysteines which normally form disulfide bonds. However, by replacing at least one of the cysteines by another amino acid (e.g. by recombinant means), it is possible to obtain an EPO where at least one of the naturally occurring cysteines comprises a free SH-group. Methods for the replacement of amino acids are known in the art (Elliott, Lorenzini, Chang, Barzilay, Delorme, 1997, Mapping of the active site of recombinant human erythropoietin, Blood, 89(2), 493-502; Boissel, Lee, Presnell, Cohen, Bunn, 1993, Erythropoietin structure-function relationships. Mutant proteins that test a model of tertiary structure, J Biol Chem., 268(21), 15983-93)).

Preferably, the EPO has the amino acid sequence of human EPO and the naturally occurring cysteines are cysteine 29 and/ or 33.

Accordingly, in a preferred embodiment, cysteine 33 is replaced by another amino acid and in step c) the modified HAS is conjugated to cysteine 29.

In a further preferred embodiment, cysteine 29 is replaced by another amino acid and in step c) the modified HAS is conjugated to cysteine 33.

In the context of the present invention, by the term "added cysteines" it is meant that the polypeptides, preferably EPO, comprise a cysteine residue which is not present in the wild type polypeptide. This can be achieved by adding (e.g. by recombinant means) a cysteine residue either at the N- or at the C-terminus of the polypeptide or by replacing (e.g. by recombinant means) a naturally-occurring amino acid by cysteine. The respective methods are known to the person skilled in the art (see above).

Preferably, the added cysteine has been added by replacing a naturally occuring amino acid by a cysteine.

In a preferred embodiment, the EPO is human EPO and the replaced amino acid residue is serine 126.

Preferably, the modified HAS is conjugated in step c) to the added cysteine.

In step b) of the method of the invention, modified HAS is provided which is capable of reacting with the EPO of step a).

In this context, the HAS may be preferably modified at its reducing end. This has the advantage that the chemical reaction can be controlled easily and that the skilled person can be sure which group of HAS is modified during the reaction. Since only one group is introduced into the HAS, crosslinking between different EPO molecules by multifunctional HAS molecules and other side reactions can be prevented.

Accordingly, the modified HAS may be capable of reacting either with
(1) at least one group linked, either directly or via a linker molecule, to sulfide groups or carbohydrate moieties of EPO,
(2) at least one carbohydrate moiety, which is preferably oxidized, and /or
(3) at least one free SH-group.

With respect to point (1) above, the modification of HAS will depend on the group linked to EPO. The underlying mechanism are known in the art. An example is given in Example 4, 2.1.

With respect to points (2) and (3) above, several methods are known in the art to modify HAS. The basic principle underlying these methods is that either a reactive group of HAS is modified in order to be capable of reacting with the carbohydrate moiety or SH-group or a linker molecule is conjugated to HAS which contains a reactive group being capable of reacting with the carbohydrate moiety or SH-group.

In case of point (2), the modified HAS may be capable of reacting with oxidized carbohydrate moieties, preferably a terminal saccharide residue, more preferably galactose, or a terminal sialic acid.

Several ways are known to modify HAS such that it is capable of reacting with an oxidized, preferably terminal saccharide residue. As mentioned above, this modification may be introduced regioselectively at the reducing end of the HES-chain. In this case, in a first step, the aldehyde group is oxidized to a lactone. The modifications include, but are not limited to the addition of hydrazide, amino (also hydroxylamino), semicarbazide or thiol functions to HAS, either directly or via a linker. These techniques are explained in further detail in Examples 2-4. Furthermore, the mechanisms per se are known in the art (see e.g. DE 196 28 705 A1; Hpoe et al., 1981, Carbohydrate Res., 91, 39; Fissekis et al., 1960, Journal of Medicinal and Pharmaceutical Chemistry, 2, 47; Frie, 1998, diploma thesis, Fachhochschule Hamburg, DE).

Within the present invention, the addition of a hydrazide or hydroxylamino function is preferred. In this case, by preferably conducting the reaction of step c) of the method of the present invention at a pH of 5.5, it is ensured that the modified HAS reacts selectively with the oxidized carbohydrate moiety of EPO without inter- or intramolecular EPO cross-linking by imine formation of lysin side chains with the oxidized saccharide residue.

In the case of point (3), also several ways are known to modify HAS such that it is capable of reacting with a free SH-group. Preferentially, this modification is introduced regioselectively at the reducing end of the HES-chain. The methods include, but are not limited to the addition of maleimide, disulfide or halogen acetamide functions to HAS. These techniques are explained in further detail in Examples 2-4.

Further details about these techniques can be obtained from Chamov et al., 1992, J. Biol. Chem., 267, 15916; Thorpe et al., 1984, Eur. J. Biochem., 140, 63; Greenfield et al., 1990, Cancer Research, 50, 6600 as well as from the literature cited in Example 2, 1.3.

Further possible functions are listed in Table 1, providing a systematic overview over possible linker molecules. Furthermore, the mechanisms per se are known in the art.

Several linker molecules which are useful in the context of the present invention are known in the art or commercially available (e.g. from Pierce, available from Perbio Science Deutschland GmbH, Bonn, Germany). Examples are given in Table 2.

In step c) of the method of the present invention, the EPO of step a) with the HAS of step b) is reacted, whereby an HAS-EPO is produced comprising one or more HAS molecules, wherein the HAS is conjugated to the EPO via a carbohydrate moiety or via a thioether.

In principle, the detailed methods how to react the EPO with the modified HAS depend on the individual modification of the EPO and /or the HAS and are known in the art (see e.g. Rose, 1994, J. Am. Chem. Soc., 116, 30, O'Shannessay and Wichek, 1990, Analytical Biochemistry, 191, 1; Thorpe et al., 1984, Eur. J. Biochem., 140, 63; Chamov et al., 1992, J. Biol. Chem. 267, 15916).

For the methods exemplified in the present invention, the details are given in Examples 2-4, especially 4.

Step c) may be performed in a reaction medium comprising at least 10 % per weight H₂O.

The reaction medium in this preferred embodiment of the method of the invention comprises at least 10 % per weight water, preferred at least 50 %, more preferred at least 80 %, e.g. 90 % or up to 100 %. The degree of organic solvents is calculated respectively. Consequently, the reaction takes place in an aqueous phase. The preferred reaction medium is water.

One advantage of this embodiment of the method of the invention is, that it is not necessary to use toxicologically critical solvents and that therefore it is not necessary to remove these solvents after the production process, in order to avoid the contamination with the solvent. Furthermore, it is not necessary to perform additional quality controls with respect to residual toxicologically critical solvents. It is preferred to use as organic solvents toxicologically not critical solvents like ethanol or propylenglycol.

Another advantage of the method of the invention is that irreversible or reversible structural changes are avoided which are induced by organic solvents. Consequently, polypeptides obtained according to the method of the invention are different from those prepared in organic solvents such as DMSO.

Furthermore, it has been surprisingly observed that the conjugation of HAS to drugs in an aqueous solution minimizes or avoids side reactions. Consequently, this embodiment of the method of the invention leads to improved products with great purity.

In the context of the present invention, the term "hydroxyalkylstarch" is used to indicate starch derivatives which have been substituted by hydroxyalkylgroups. In this context, the alkyl group may be substituted. Preferably, the hydroxyalkyl contains 2-10 carbon atoms, more preferably 2-4 carbon atoms. "Hydroxyalkylstarch" therefore preferably comprises hydroxyethylstarch, hydroxypropylstarch and hydroxybutylstarch, wherein hydroxyethylstarch and hydroxypropylstarch are preferred.

The hydroxyalkylgroup(s) of HAS contain at least one OH-group.

Hydroxyethylstarch (HES) is most preferred for all embodiments of the present invention.

The expression "hydroxyalkylstarch" also includes derivatives wherein the alkyl group is mono- or polysubstituted. In this context, it is preferred that the alkyl group is substituted with an halogen, especially flourine, or with an aryl group, provided that the HAS remains water soluble. Furthermore, the terminal hydroxy group of hydroxyalkyl may be esterified or etherified. In addition, the alkyl group of the hydroxyalkylstarch may be linear or branched.

Furthermore, instead of alkyl, also linear or branched substituted or unsubstituted alkylene groups may be used.

In the context of the present invention, hydroxyethylstarch may have a mean molecular weight (weight mean) of 1-300 kDa, wherein a mean molecular weight of 5-100 kDa is more preferred. Hydroxyethylstarch may further exhibit a molar degree of substitution of 0.1 to 0.8 and a ratio between C₂:C₆-substitution in the range of 2-20, with respect to the hydroxyethylgroups.

The HAS-EPO produced by the method of the invention can be purified and characterized as follows:

Isolation of the HAS-EPO can be performed by using known procedures for the purification of natural and recombinant EPO (e.g.size exclusion chromatography, ion-exchange chromatography, RP-HPLC, hydroxyapatite chromatography, hydrophobic interaction chromatography or combinations thereof ).

The covalent attachment of HAS to the EPO polypetide can be verified by carbohydrate compositional analysis after hydrolysis of the modified protein (ratio of hydroxyethylglucose and mannose present on the three N-glycosylation sites of EPO).

Demonstration of HAS modification at N-linked oligosaccharides of EPO can be accomplished by removal of the HAS modified N-glycans and observation of the predicted shift to higher mobility in SDS-PAGE +/- Western Blotting analysis.

HAS modification of EPO at cysteine residues can be demonstrated by the failure to detect the corresponding proteolytic Cys-peptide in RP-HPLC and MALDI/TOF-MS in the proteolytic fragments of the HAS-modified product (Zhou et al., 1998, Application of capillary electrophoresis, liquid chromatography, electrospray-mass spectrometry and matrix-assisted laserdesorption/ionization - time of flight - mass spectrometry to the characterization of recombinant human erythropoietin. Electrophoresis, 19(13), 2348-55). The isolation of the HAS-containing fraction after proteolytic digestion of the Cys-modified EPO enables the verification in this fraction of the corresponding peptide by conventional amino acid compositional analysis.

All embodiments disclosed above with respect of the HAS-EPO of the invention concerning properties of EPO or HAS apply also to the method of the invention for preparing a HAS-EPO.

The invention further relates to a HAS-EPO, obtainable by the method of the invention. Preferably, this HAS-EPO has the features as defined for the above HAS-EPO of the invention.

The invention further relates to a HAS-EPO according to the invention for use in a method for treatment of the human or animal body.

Furthermore, the present invention relates to a pharmaceutical composition comprising the HAS-EPO of the invention. In a preferred embodiment, the pharmaceutical composition comprises further at least one pharmaceutically acceptable diluent, adjuvant and/or carrier useful in erythropoietin therapy.

A "therapeutically effective amount" as used herein refers to that amount which provides therapeutic effect for a given condition and administration regimen. The administration of erythropoietin isoforms is preferably by parenteral routes. The specific route chosen will depend upon the condition being treated. The administration of erythropoietin isoforms is preferably done as part of a formulation containing a suitable carrier, such as human serum albumin, a suitable diluent, such as a buffered saline solution, and/or a suitable adjuvant. The required dosage will be in amounts sufficient to raise the hematocrit of patients and will vary depending upon the severity of the condition being treated, the method of administration used and the like.

The object of the treatment with the pharmaceutical composition of the invention is preferably an increase of the hemoglobin value of more than 6.8 mmol/l in the blood. For this, the pharmaceutical composition may be administered in a way that the hemoglobin value increases between 0.6 mmol/l and 1.6 mmol/l per week. If the hemoglobin value exceeds 8.7 mmol/l, the therapy should be preferably interrupted until the hemoglobin value is below 8.1 mmol/l.

The composition of the invention is preferably used in a formulation suitable for subcutaneous or intravenous or parenteral injection. For this, suitable excipients and carriers are e.g. sodium dihydrogen phosphate, disodium hydrogen phosphate, sodium chlorate, polysorbate 80, HSA and water for injection. The composition may be administered three times a week, preferably two times a week, more preferably once a week, and most preferably every two weeks.

Preferably, the pharmaceutical composition is administered in an amount of 0.01-10 µg/kg body weight of the patient, more preferably 0,1 to 5 µg/kg, 0,1 to 1 µg/kg, or 0.2-0.9 µg/kg, most preferably 0.3-0.7 µg/kg, and most preferred 0.4-0.6 µg/kg body weight.

In general, preferably between 10 µg and 200 µg, preferably between 15 µg and 100 µg are administered per dosis.

The invention further relates to the use of a HAS-EPO of the invention for the preparation of a medicament for the treatment of anemic disorders or hematopoietic dysfunction disorders or diseases related hereto.

According to a further aspect of the present invention, the problem is solved by a hydroxyalkylstarch (HAS)-polypeptide-conjugate (HAS-polypeptide) comprising one or more HAS molecules, wherein each HAS is conjugated to the polypeptide via
a) a carbohydrate moiety; or
b) a thioether.

The HAS-polypeptide of the invention has the advantage that it exhibits an improved biological stability when compared to the polypeptide before conjugation. This is mainly due to the fact that HAS-polypeptide is less or not recognized by the removal systems of the liver and kidney and therefore persists in the circulatory system for a longer period of time. Furthermore, since the HAS is attached site-specifically, the risk of destroying the in vivo biological activity of the polypeptide by conjugation of HAS to the polypeptide is minimized.

The HAS-polypeptide of the invention has mainly two components, namely the polypeptide and the hydroxyalkylstarch (HAS) linked thereto.

The polypeptide can be of any human or animal source. In a preferred embodiment, the polypeptide is of human source.

The polypeptide may be a cytokine, especially erythropoietin, an interleukin, especially interleukin-2, IFN-β, IFN-alpha, CSF, interleukin-6 and therapeutic antibodies.

With respect to erythropoietin, all embodiments disclosed above also apply here.

Preferably, the polypeptide is recombinantly produced. This includes the production in eukaryotic or prokaryotic cells, preferably mammalian, insect, yeast, bacterial cells or in any other cell type which is convenient for the recombinant production of the polypeptide. Furthermore, the polypeptide may be expressed in transgenic animals (e.g. in body fluids like milk, blood, etc.), in eggs of transgenic birds, especially poultry, preferred chicken, or in transgenic plants.

The recombinant production of a polypeptide is known in the art. In general, this includes the transfection of host cells with an appropriate expression vector, the cultivation of the host cells under conditions which enable the production of the polypeptide and the purification of the polypeptide from the host cells. For detailled information see e.g. Krystal, Pankratz, Farber, Smart, 1986, Purification of human erythropoietin to homogeneity by a rapid five-step procedure, Blood, 67(1), 71-9; Quelle, Caslake, Burkert, Wojchowski, 1989, High-level expression and purification of a recombinant human erythropoietin produced using a baculovirus vector, Blood, 74(2), 652-7; EP 640 619 B and EP 668 351 B1.

The polypeptide may comprise one or more carbohydrate side chains attached to the polyppetide via N- and/ or O-linked glycosylation, i.e. the polypeptide is glycosylated. Usually, when a polypeptide is produced in eukaryotic cells, the polypeptide is posttranslationally glycosylated. Consequently, the carbohydrate side chains may have been attached to the polypeptide during biosynthesis in mammalian, especially human, insect or yeast cells.

The HAS may be directly conjugated to the polypeptide or, alternatively, via a linker molecule. The nature of the linker molecule depends on the way how the HAS is linked to the polypeptide. Several linkers are commercially available (e.g. from Pierce, see above). The nature of the linker and its purpose are described in detail below in the section concerning the method for the production of HES-polypeptide is discussed.

According to a preferred embodiment of the HAS-polypeptide conjugate of the invention, the HAS is conjugated to the polypeptide via a carbohydrate moiety.

In the context of the present invention, the term "carbohydrate moiety" refers to hydroxyaldehydes or hydroxyketones as well as to chemical modifications thereof (see Römpp Chemielexikon, 1990, Thieme Verlag Stuttgart, Germany, 9^{th} edition, 9, 2281-2285 and the literature cited therein). Furthermore, it also refers to derivatives of naturally occuring carbohydrate moieties like glucose, galactose, mannose, sialic acid, and the like. The term also includes chemically oxidized naturally occuring carbohydrate moieties wherein the ring structure has been opened.

The carbohydrate moiety may be linked directly to the polypeptide backbone. Preferably, the carbohydrate moiety is part of a carbohydrate side chain. In this case, further carbohydrate moieties may be present between the carbohydrate moiety to which HAS is linked and the polypeptide backbone. More preferably, the carbohydrate moiety is the terminal moiety of the carbohydrate side chain.

In a more preferred embodiment, the HAS is conjugated to a galactose residue of the carbohydrate side chains, preferably the terminal galactose residue of the carbohydrate side chain. This galactose residue can be made available for conjugation by removal of terminal sialic acids, followed by oxidation (see below).

In a further more preferred embodiment, the HAS is conjugated to a sialic acid residue of the carbohydrate side chains, preferably the terminal sialic acid residue of the carbohydrate side chain.

Furthermore, the HAS may be conjugated to the polypeptide via a thioether. As explained in detail below, the S atom can be derived from any SH group attached to the polypeptide, both naturally or non naturally occurring.

In a preferred embodiment, the S atom may be derived from a SH group which has been introduced in an oxidized carbohydrate moiety of HES, preferably an oxidized carbohydrate moiety which is part of a carbohydrate side chain of the polypeptide (see below).

Preferably, the S atom in the thioether is derived from a naturally-occurring cysteine or from an added cysteine.

In the context of the present invention, by the term "added cysteines" it is meant that the polypeptides comprise a cysteine residue which is not present in the wild-type polypeptide.

In the context of this aspect of the invention, the cysteine may be an additional amino acid added at the N- or C-terminal end of the polypeptide.

Furthermore, the added cysteine may have been added by replacing a naturally occuring amino acid by a cysteine.

The second component of the HAS-polypeptide is HAS.

In the context of the present invention, the term "hydroxyalkylstarch" is used to indicate starch derivatives which have been substituted by hydroxyalkylgroups. In this context, the alkyl group may be substituted. Preferably, the hydroxyalkyl contains 2-10 carbon atoms, more preferably 2-4 carbon atoms. "Hydroxyalkylstarch" therefore preferably comprises hydroxyethylstarch, hydroxypropylstarch and hydroxybutylstarch, wherein hydroxyethylstarch and hydroxypropylstarch are preferred.

The hydroxyalkylgroup(s) of HAS contain at least one OH-group.

The expression "hydroxyalkylstarch" also includes derivatives wherein the alkyl group is mono- or polysubstituted. In this context, it is preferred that the alkyl group is substituted with an halogen, especially flourine, or with an aryl group, provided that the HAS remains water soluble. Furthermore, the terminal hydroxy group of hydroxyalkyl may be esterified or etherified. In addition, the alkyl group of the hydroxyalkylstarch may be linear or branched.

Furthermore, instead of alkyl, also linear or branched substituted or unsubstituted alkene groups may be used.

Hydroxyethylstarch (HES) is most preferred for all embodiments of the present invention.

In the context of the present invention, hydroxyethylstarch may have a mean molecular weight (weight mean) of 1-300 kDa, wherein a mean molecular weight of 5-100 kDa is more preferred. Hydroxyethylstarch can further exhibit a molar degree of substitution of 0.1 to 0.8 and a ratio between C₂:C₆-substitution in the range of 2-20, with respect to the hydroxyethylgroups.

The HAS-polypeptide may comprise 1-12, preferably 1-9, 1-6 or 1-3, most preferred 1-4 HAS molecules per polypeptide molecule. The number of HAS-molecules per polypeptide molecule can be determined by quantitative carbohydrate compositional analysis using GC-MS after hydrolysis of the product and derivatisation of the resulting monosaccharides (Chaplin and Kennedy, 1986, Carbohydrate Analysis (eds.): a practical approach ed., Chapter 1. Monosaccharides page 1-36; Chapter 2. Oligosaccharides page 37-53; Chapter 3. Neutral Polysaccharides; 55-96; IRL Press Practical approach series (ISBN 0-947946-44-3).

All embodiments disclosed below with respect of the method of the invention to produce a HAS-polypeptide concerning properties of the polypeptide or HAS apply also to the HAS-polypeptide of the invention. Furthermore, all embodiments disclosed above with respect to HAS-EPO or the preparation thereof which relate to peptides in general or to HAS apply also to the HAS-polypeptide of the invention.

Hydroxyalkylstarch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially of acetic acid, In this context, acetylstarch, butylstarch or propylstarch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

In a further aspect, the present invention relates to a method for the production of a hydroxyalkylstarch (HAS)-polypeptide-conjugate (HAS-polypeptide), comprising the steps of:
a) providing a polypeptide being capable of reacting with modified HAS,
b) providing modified HAS being capable of reacting with the polypeptide of step a), and
c) reacting the polypeptide of step a) with the HAS of step b), whereby an HAS-polypeptide is produced comprising one or more HAS molecules, wherein the HAS is conjugated to the polypeptide via
   i) a carbohydrate moiety; or
   ii) a thioether.

The method of the invention has the advantage that a HAS-polypeptide conjugate is produced which exhibits a high biological activity. Furthermore, the method of the invention has the advantage that an effective polypetide derivative can be produced at reduced cost since the method does not comprise extensive and time consuming purification steps resulting in low final yield.

Accordingly, in the first step of the method of the invention, a polypeptide is provided which is capable of reacting with modified HAS.

As used in the present invention, the term "providing" has to be interpreted in the way that after the respective step a molecule (in step a) a polypeptide, in step b) HAS) with the desired properties is available.

In the case of step a), this includes the purification of the polypeptide from natural sources as well as the recombinant production in host cells or organism, and, if necessary, the modification of the polypeptide so obtained.

With respect to the polypeptide being the starting material of the present invention, the same applies as for the erythropoietin being part of the HAS-polypeptide conjugate of the invention. In this context, the preferred embodiments disclosed above apply also for the method of the invention.

Preferably, the polypeptide is recombinantly produced. This includes the production in eukaryotic or prokaryotic cells, preferably mammalian, insect, yeast, bacterial cells or in any other cell type which is convenient for the recombinant production of the polypeptide. Furthermore, the polypeptide may be expressed in transgenic animals (e.g. in body fluids like milk, blood, etc.), in eggs of transgenic birds, especially poultry, preferred chicken, or in transgenic plants.

The recombinant production of a polypeptide is known in the art. In general, this includes the transfection of host cells with an appropriate expression vector, the cultivation of the host cells under conditions which enable the production of the polypeptide and the purification of the polypeptide from the host cells (Krystal, Pankratz, Farber, Smart, 1986, Purification of human erythropoietin to homogeneity by a rapid five-step procedure, Blood, 67(1), 71-9; Quelle, Caslake, Burkert, Wojchowski, 1989, High-level expression and purification of a recombinant human erythropoietin produced using a baculovirus vector, Blood, 74(2), 652-7; EP 640 619 B1 and EP 668 351 B1).

The polypeptide may comprise one or more carbohydrate side chains attached to the polypeptide via N- and/ or O-linked glycosylation, i.e. the polypeptide is glycosylated. Unsually, when the polypeptide is produced in eukaryotic cells, the polypeptide is posttranslationally glycosylated. Consequently, the carbohydrate side chains may have been attached to the polypeptide during production in mammalian, especially human, insect or yeast cells, wherein the cells may be those of a transgenic animal or plant (see above).

These carbohydrate side chains may have been chemically or enzymatically modified after the expression in the appropriate cells, e.g. by removing or adding one or more carbohydrate moieties (see e.g. Dittmar, Conradt, Hauser, Hofer, Lindenmaier, 1989, Advances in Protein design; Bloecker, Collins, Schmidt, and Schomburg eds., GBF-Monographs, 12, 231-246, VCH Publishers, Weinheim, New York, Cambridge)

It is the object of the method of the invention to provide an HAS-polypeptide comprising one or more HAS molecules wherein the HAS is conjugated to the polypeptide via a carbohydrate moiety (i) or via a thioether (ii). Consequently, the polypeptide provided in step a) should have the properties that a conjugation via a carbohydrate moiety and/ or via a thioether is possible. Therefore the polypeptide after step a) may preferably contain either
(1) at least one reactive group linked, either directly or via a linker molecule, to sulfide groups or carbohydrate moieties, which is capable to react with HES or modified HES,
(2) at least one carbohydrate moiety to which modified HAS can be conjugated, and/or
(3) at least one free SH-group.

With respect to possibility (1) above, the polypeptide of step a) is preferably obtainable by conjugating an appropriate linker molecule to the SH-group(s) or carbohydrate moieties of the polypeptide. An example for such a modified polypeptide is provided in Example 4, 2.1. It is important to ensure that the addition of the linker molecule does not damage the polypeptide. However, this is known to the person skilled in the art.

With respect to possibility (2) above, in a preferred embodiment, the modified HAS is conjugated to the polypeptide via a carbohydrate moiety.

The carbohydrate moiety may be linked directly to the polypeptide backbone. Preferably, the carbohydrate moiety is part of a carbohydrate side chain. In this case, further carbohydrate moieties may be present between the carbohydrate moiety to which HAS is linked and the polypeptide backbone. More preferably, the carbohydrate moiety is the terminal moiety of the carbohydrate side chain.

Consequently, in a preferred embodiment, the modified HAS is attached (via a linker or not, see below) to carbohydrate chains linked to N- and/ or O-glycosylation sites of the polypeptide.

However, it is also included within the present invention that the polypeptide contains (a) further carbohydrate moiet(y)ies to which the modified HAS is conjugated. Techniques for attaching carbohydrate moieties to polypeptides, either enzymatically or by genetic engineering, followed by expression in appropriate cells, are known in the art (Berger, Greber, Mosbach, 1986, Galactosyltransferase-dependent sialylation of complex and endo-N-acetylglucosaminidase H-treated core N-glycans in vitro, FEBS Lett., 203(1), 64-8; Dittmar, Conradt, Hauser, Hofer, Lindenmaier, 1989, Advances in Protein design; Bloecker, Collins, Schmidt, and Schomburg eds., GBF-Monographs, 12, 231-246, VCH Publishers, Weinheim, New York, Cambridge).

In a preferred embodiment of the method of the invention, the carbohydrate moiety is oxidized in order to be able to react with the modified HAS. This oxidation can be performed either chemically or enzymatically.

Methods for the chemical oxidation of carbohydrate moieties of polypeptides are known in the art and include the treatment with perjodate (Chamow et al., 1992, J. Biol. Chem., 267, 15916-15922).

By chemically oxidizing, it is principally possible to oxidize any carbohydrate moiety, being terminally positioned or not. However, by choosing mild conditions (1 mM periodate, 0 °C in contrast to harsh conditions: 10 mM periodate 1h at room temperature), it is possible to preferably oxidize the terminal carbohydrate moiety, e.g. sialic acid or galactose, of a carbohydrate side chain.

Alternatively, the carbohydrate moiety may be oxidized enzymatically. Enzymes for the oxidation of the individual carbohydrate moieties are known in the art, e.g. in the case of galactose the enzyme is galactose oxidase.

If it is intended to oxidize terminal galactose moieties, it will be eventually necessary to remove terminal sialic acids (partially or completely) if the polypeptide has been produced in cells capable of attaching sialic acids to carbohydrate chains, e.g. in mammalian cells or in cells which have been genetically modified to be capable of attaching sialic acids to carbohydrate chains. Chemical or enzymatic methods for the removal of sialic acids are known in the art (Chaplin and Kennedy (eds.), 1996, Carbohydrate Analysis: a practical approach, especially Chapter 5 Montreuill, Glycoproteins, pages 175-177; IRL Press Practical approach series (ISBN 0-947946-44-3)).

However, it is also included within the present invention that the carbohydrate moiety to which the modified HAS is to be attached is attached to the polypeptide within step a). In the case it is desired to attach galactose, this can be achieved by the means of galactose transferase. The methods are known in the art (Berger, Greber, Mosbach, 1986, Galactosyltransferase-dependent sialylation of complex and endo-N-acetylglucosaminidase H-treated core N-glycans in vitro, FEBS Lett., 203(1), 64-8).

In a most preferred embodiment, in step a) the polypeptide is modified by oxidizing at least one terminal saccharide unit, preferably galactose, of the one or more carbohydrate side chains of the polypeptide, preferably after partial or complete (enzymatic and/ or chemical) removal of the terminal sialic acid, if necessary (see above).

Consequently, preferably the modified HAS is conjugated to the oxidized terminal saccharide unit of the carbohydrate chain, preferably galactose.

In a further preferred embodiment (see point (3) above), the polypeptide comprises at least one free SH-group.

According to a preferred embodiment, the free SH-group is part of a naturally-occurring cysteine or of an added cysteine.

Methods for the replacement of amino acids are known in the art (Elliott, Lorenzini, Chang, Barzilay, Delorme, 1997,Mapping of the active site of recombinant human erythropoietin, Blood, 89(2), 493-502; Boissel, Lee, Presnell, Cohen, Bunn, 1993, Erythropoietin structure-function relationships. Mutant proteins that test a model of tertiary structure, J Biol Chem., 268(21), 15983-93)).

In the context of the present invention, by the term "added cysteines" it is meant that the polypeptides comprise a cysteine residue which is not present in the wild type polypeptide. This can be achieved by adding (e.g. by recombinant means) a cysteine residue either at the N- or at the C-terminus of the polypeptide or by replacing (e.g. by recombinant means) a naturally-occurring amino acid by cysteine. The respective methods are known to the person skilled in the art (see above).

Preferably, the added cysteine has been added by replacing a naturally occuring amino acid by a cysteine.

Preferably, the modified HAS is conjugated in step c) to the added cysteine.

In step b) of the method of the invention, modified HAS is provided which is capable of reacting with the polypeptide of step a).

In this context, the HAS may be preferably modified at its reducing end. This has the advantage that the chemical reaction can be controlled easily and that the skilled person can be sure which group of HAS is modified during the reaction. Since only one group is introduced into the HAS, crosslinking between different polypeptide molecules by multifunctional HAS molecules and other side reactions can be prevented.

Accordingly, the modified HAS may be capable of reacting either with
(1) at least one group linked, either directly or via a linker molecule, to sulfide groups or carbohydrate moieties of the polypeptide,
(2) at least one carbohydrate moiety, which is preferably oxidized, and /or
(3) at least one free SH-group.

With respect to point (1) above, the modification of HAS will depend on the group linked to the polypeptide. The underlying mechanism are known in the art. An example is given in Example 4, 2.1.

With respect to points (2) and (3) above, several methods are known in the art to modify HAS. The basic principle underlying these methods is that either a reactive group of HAS is modified in order to be capable of reacting with the carbohydrate moiety or SH-group or a linker molecule is conjugated to HAS which contains a reactive group being capable of reacting with the carbohydrate moiety or SH-group.

In case of point (2), the modified HAS may be capable of reacting with oxidized carbohydrate moieties, preferably a terminal saccharide residue, more preferably galactose, or with a terminal sialic acid.

Several ways are known to modify HAS such that it is capable of reacting with an oxidized, preferably terminal saccharide residue. As mentioned above, this modification may be introduced regioselectively at the reducing end of the HES-chain. In this case, in a first step, the aldehyde group is oxidized to a lactone. The modifications include, but are not limited to the addition of hydrazide, amino (also hydroxylamino), semicarbazide or thiol functions to HAS, either directly or via a linker. These techniques are explained in further detail in Examples 2-4. Furthermore, the mechanisms per se are known in the art (see e.g. DE 196 28 705 A1; Hpoe et al., 1981, Carbohydrate Res., 91, 39; Fissekis et al., 1960, Journal of Medicinal and Pharmaceutical Chemistry, 2, 47; Frie, 1998, diploma thesis, Fachhochschule Hamburg, DE).

Within the present invention, the addition of a hydrazide or hydroxylamino function is preferred. In this case, by preferably conducting the reaction of step c) of the method of the present invention at a pH of 5.5, it is ensured that the modified HAS reacts selectively with the oxidized carbohydrate moiety of the polypeptide without inter- or intramolecular polypeptide cross-linking by imine formation of lysine side chains with the oxidized saccharide residue.

In the case of point (3), also several ways are known to modify HAS such that it is capable of reacting with a free SH-group. Preferentially, this modification is introduced regioselectively at the reducing end of the HES-chain. The methods include, but are not limited to the addition of maleimide, disulfide or halogen acetamide functions to HAS. These techniques are explained in further detail in Examples 2-4

Further details about these techniques can be obtained from Chamov et al., 1992, J. Biol. Chem., 267, 15916; Thorpe et al., 1984, Eur. J. Biochem., 140, 63; Greenfield et al., 1990, Cancer Research, 50, 6600 as well as from the literature cited in Example 2, 1.3.

Further possible functions are listed in Table 1, providing a systematic overview over possible linker molecules. Furthermore, the mechanisms per se are known in the art.

Several linker molecules which are useful in the context of the present invention are known in the art or commercially available (e.g. from Pierce, available from Perbio Science Deutschland GmbH, Bonn, Germany).

In step c) of the method of the present invention, the polypeptide of step a) with the HAS of step b) is reacted, whereby an HAS-polypeptide is produced comprising one or more HAS molecules wherein the HAS is conjugated to the polypeptide via a carbohydrate moiety or via a thioether.

In principle, the detailed methods how to react the polypeptide with the modified HAS depend on the individual modification of the polypeptide and /or the HAS and are known in the art (see e.g. Rose, 1994, J. Am. Chem. Soc., 116, 30; O'Shannessay and Wichek, 1990, Analytical Biochemistry, 191, 1; Thorpe et al., 1984, Eur. J. Biochem., 140, 63; Chamov et al., 1992, J. Biol. Chem., 267, 15916).

For the methods exemplified in the present invention, the details are given in Examples 2-4, especially 4.

Step c) may be performed in a reaction medium comprising at least 10 % per weight H₂O.

The reaction medium in this preferred embodiment of the method of the invention comprises at least 10 % per weight water, preferred at least 50 %, more preferred at least 80 %, e.g. 90 % or up to 100 %. The degree of organic solvents is calculated respectively. Consequently, the reaction takes place in an aqueous phase. The preferred reaction medium is water.

One advantage of this embodiment of the method of the invention is, that it is not necessary to use toxicologically critical solvents and that therefore it is not necessary to remove these solvents after the production process, in order to avoid the contamination with the solvent. Furthermore, it is not necessary to perform additional quality controls with respect to residual toxicologically critical solvents. It is preferred to use as organic solvents toxicologically not critical solvents like ethanol or propylenglycol.

Another advantage of the method of the invention is that irreversible or reversible structural changes are avoided which are induced by organic solvents. Consequently, polypeptides obtained according to the method of the invention are different from those prepared in organic solvents such as DMSO.

Furthermore, it has been surprisingly observed that the conjugation of HAS to drugs in an aqueous solution avoids side reactions. Consequently, this embodiment of the method of the invention leads to improved products with great purity.

In the context of the present invention, the term "hydroxyalkylstarch" is used to indicate starch derivatives which have been substituted by hydroxyalkylgroups. In this context, the alkyl group may be substituted. Preferably, the hydroxyalkyl contains 2-10 carbon atoms, more preferably 2-4 carbon atoms. "Hydroxyalkylstarch" therefore preferably comprises hydroxyethylstarch, hydroxypropylstarch and hydroxybutylstarch, wherein hydroxyethylstarch and hydroxypropylstarch are preferred.

The hydroxyalkylgroup(s) of HAS contain at least one OH-group.

Hydroxyethylstarch (HES) is most preferred for all embodiments of the present invention.

The expression "hydroxyalkylstarch" also includes derivatives wherein the alkyl group is mono- or polysubstituted. In this context, it is preferred that the alkyl group is substituted with an halogen, especially flourine, or with an aryl group, provided that the HAS remains water soluble. Furthermore, the terminal hydroxy group of hydroxyalkyl may be esterified or etherified. In addition, the alkyl group of the hydroxyalkylstarch may be linear or branched.

Furthermore, instead of alkyl, also linear or branched substituted or unsubstituted alkylene groups may be used.

In the context of the present invention, hydroxyethylstarch may have a mean molecular weight (weight mean) of 1-300 kDa, wherein a mean molecular weight of 5-100 kDa is more preferred. Hydroxyethylstarch may further exhibit a molar degree of substitution of 0.1 to 0.8 and a ratio between C₂:C₆-substitution in the range of 2-20, with respect to the hydroxyethylgroups.

The HAS-polypepetide produced by the method of the invention can be purified and characterized as follows:

Isolation of the HAS-polypeptide can be performed by using known procedures for the purification of natural and recombinant polypeptides (e.g. size exclusion chromatography, ion-exchange chromatography, RP-HPLC, hydroxyapatite chromatography, hydrophobic interaction chromatography or combinations thereof ).

The covalent attachment of HAS to the polypetide can be verified by carbohydrate compositional analysis after hydrolysis of the modified protein.

Demonstration of HAS modification at N-linked oligosaccharides of the polypeptide can be accomplished by removal of the HAS modified N-glycans and observation of the predicted shift to higher mobility in SDS-PAGE +/- Western Blotting analysis.

HAS modification of the polypeptide at cysteine residues can be demonstrated by the failure to detect the corresponding proteolytic Cys-peptide in RP-HPLC and MALDI/TOF-MS in the proteolytic fragments of the HAS-modified product (Zhou et al., 1998, Application of capillary electrophoresis, liquid chromatography, electrospray-mass spectrometry and matrix-assisted laserdesorption/ionization - time of flight - mass spectrometry to the characterization of recombinant human erythropoietin, Electrophoresis, 19(13), 2348-55). The isolation of the HAS-containing fraction after proteolytic digestion of the Cys-modified polypeptide enables the verification in this fraction of the corresponding peptide by conventional amino acid compositional analysis.

All embodiments disclosed above with respect of the HAS-polypeptide of the invention concerning properties of the polypeptide or HAS apply also to the method of the invention for the production of a HAS-polypeptide conjugate. Furthermore, all embodiments disclosed above with respect to HAS-EPO or the preparation thereof which relate to peptides in general or to HAS apply also to the method of the invention for the production of a HAS-polypeptide conjugate.

The invention further relates to a HAS-polypeptide, obtainable by the method of the invention. Preferably, this HAS-polypeptide has the features as defined for the above HAS-polypeptide of the invention.

The invention further relates to a HAS-polypeptide according to the invention for use in a method for treatment of the human or animal body.

Furthermore, the present invention relates to a pharmaceutical composition comprising the HAS-polypeptide of the invention. In a preferred embodiment, the pharmaceutical composition comprises further at least one pharmaceutically acceptable diluent, adjuvant and/or carrier useful in erythropoietin therapy.

The invention further relates to the use of a HAS-polypeptide of the invention for the preparation of a medicament for the treatment of anemic disorders or hematopoietic dysfunction disorders or diseases related hereto.

The invention is further illustrated by the following figures, tables and examples, which are in no way intended to restrict the scope of the present invention.

### Short description of the Figures

### Figure 1

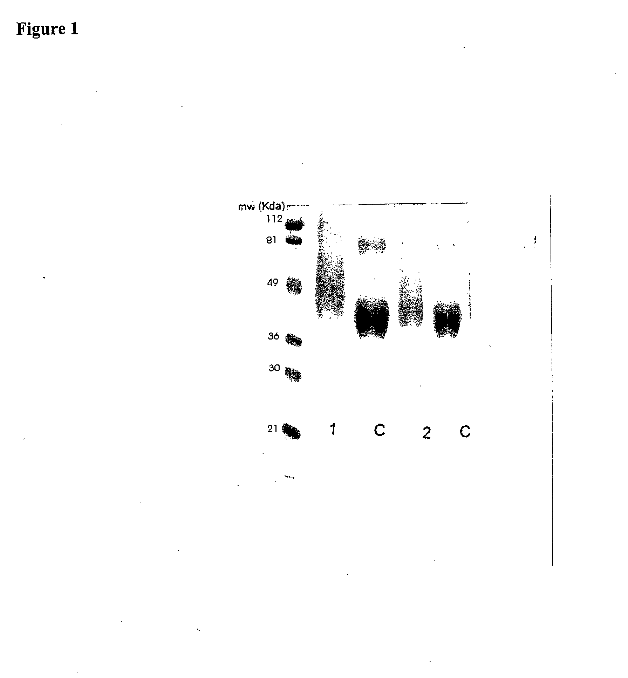

Figure 1 shows an SDS page analysis of two HES-EPO conjugates
- mw:: marker
- Lane 1:: HES-EPO produced according to example protocol 8: EPO is conjugated to hydrazido-HES 12KD L
- Lane 2:: HES-EPO produced according to example protocol 9 : EPO is conjugated to hydroxylamino HES 12 KD K
- C:: control (unconjugated EPO); the upper band represents EPO dimer

### Figure 2

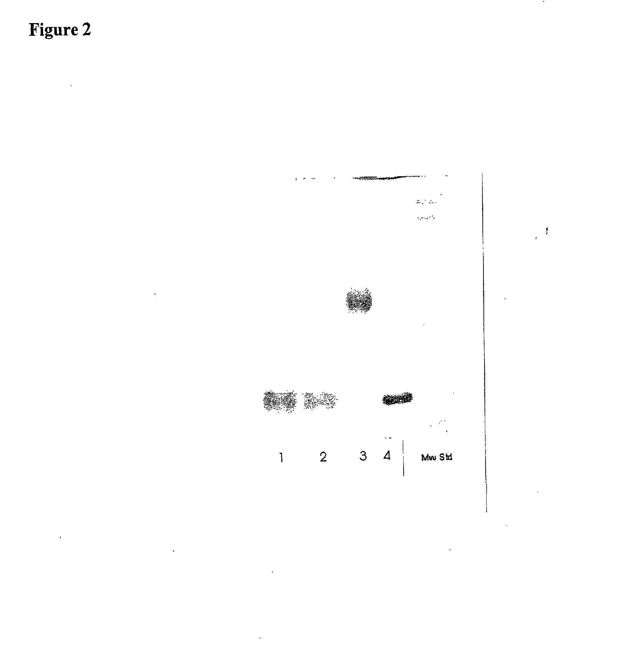

Figure 2 demonstrates that the HES is conjugated to a carbohydrate moiety of a carbohydrate side chain by showing a digestion of HAS modified EPO forms with polyppetide N-glycosidase
- Lane 1:: HES-EPO produced according to example protocol 8 after digestion with N-glycosidase
- Lane 2:: HES-EPO produced according to example protocol 9 after digestion with N-glycosidase
- Lane 3:: BRP EPO standard
- Lane 4:: BRP EPO standard after digestion with N-glycosidase
- mw:: marker (Bio-Rad SDS-PAGE Standards Low range Catalog No 161-0305, Bio-Rad Laboratories, Hercules, CA, USA)

### Examples

### Example 1

### Production of recombinant EPO

### A) Production in mammalian cells

Recombinant EPO was produced in CHO cells as follows

A plasmid harbouring the human EPO cDNA was cloned into the eukaryotic expression vector (pCR3 and named afterwards pCREPO). Site directed mutagenesis was performed using standard procedures as described (Grabenhorst, Nimtz, Costa et al., 1998, In vivo specificity of human alpha 1,3/4-fucosyltransferases III-VII in the biosynthesis of Lewis(x) and sialyl Lewis(x) motifs on complex-type N-glycans -Coexpression studies from BHK-21 cells together with human beta-trace protein, J. Biol. Chem., 273(47), 30985-30994).

CHO cells stably expressing human EPO or amino acid variants (e.g. Cys-29→Ser/Ala, or Cys-33→Ser/Ala , Ser-126→Ala etc.) thereof were generated with the calcium phosphate precipitation method and selected with G418-sulfate as described (Grabenhorst et al.). Three days after transfection, the cells were sub-cultivated 1:5 and selected in DMEM containing 10% FBS and 1.5 g/liter G418 sulfate.

Using this selection procedure, usually 100-500 clones survived and where propagated in selection medium for a further time period of 2-3 weeks. Cell culture supematants of confluently growing monolayers were then analyzed for EPO expression levels by Western blot analysis and by IEF/Western Blot analysis.

EPO was produced from stable subclones in spinner flasks or in 21 perfusion reactors. Different glycoforms of EPO with different amounts of NeuAc (e.g. 2-8, 4-10, 8-12 NeuAc residues) were isolated according to published protocols using combinations various chromatographic procedures as described below.

### Literature:

Grabenhorst, Conradt, 1999, The cytoplasmic, transmembrane, and stem regions of glycosyltransferases specify their in vivo functional sublocalization and stability inthe Golgi., J Biol Chem., 274(51), 36107-16; Grabenhorst, Schlenke, Pohl, Nimtz, Conradt, 1999, Genetic engineering of recombinant glycoproteins and the glycosylation pathway in mammalian host cells, Glycoconj J., 16(2), 81-97; Mueller, Schlenke, Nimtz, Conradt, Hauser, 1999, Recombinant glycoprotein product quality in proliferation-controlled BHK-21 cells, Biotechnology and bioengineering, 65(5), 529-536; Schlenke, Grabenhorst, Nimtz, Conradt, 1999, Construction and characterization of stably transfected BHK-21 cells with human-type sialylation characteristic, Cytotechnology, 30(1-3), 17-25.

### B) Production in insect cells

Recombinant human EPO was produced from insect cell lines SF9 and SF 21 after infection of cells with recombinant baculovirus vector containing the human EPO cDNA under control of the polyhedrin promoter as described in the literature.

Cells grown in serum-free culture medium were infected at cell density of 2x 10⁶ or X10⁷ cells per mL and EPO titers were determined every day in the cell culture supernatants. EPO was purified by Blue sepharose chromatography, ion-exchange chromatography on Q-Sepharose and finally RP-HPLC on C₄-Phase.

Purity of the product was checked by SDS-PAGE and N-terminal sequencing. Detailled carbohydrate structural analysis (N- and O-glycosylation) was performed according to published procedures.

### Literature:

Grabenhorst, Hofer, Nimtz, Jager, Conradt, 1993, Biosynthesis and secretion of human interleukin 2 glycoprotein variants from baculovirus-infected Sf21 cells. Characterization ofpolypeptides and posttranslational modifications, Eur J Biochem., 215(1), 189-97; Quelle, Caslake, Burkert, Wojchowski, 1989, High-level expression and purification of a recombinant human erythropoietin produced using a baculovirus vector, Blood, 74(2), 652-7

### Example 2

### Formation of reactive HES derivatives

### 1. SH-reactive HES

### 1.1 Reaction of EMCH with Oxo-HES12KD to form SH-reactive HES12KD B

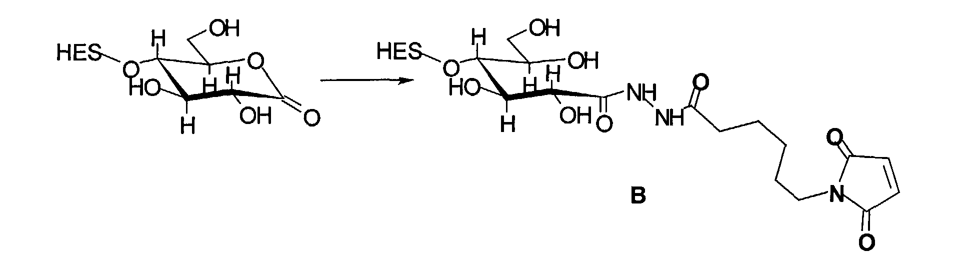

0.144 g (0.012 mmol) of Oxo-HES12KD (Fresenius German Patent DE 196 28 705 A1) were dissolved in 0.3 mL absolute dimethyl sulfoxide (DMSO) and were added dropwise under nitrogen to a mixture of 34 mg (0.15 mmol) EMCH (Perbio Science, Deutschland GmbH, Bonn, Germany) in 1.5 mL DMSO. After stirring for 19 h at 60°C the reaction mixture was added to 16 mL of a 1:1 mixture of ethanol and acetone. The precipitate was collected by centrifugation, redissolved in 3 mL DMSO and again precipitated as described. The SH-reactiv-HES12KD **B** was obtained by centrifugation and drying in vaccuo. The conjugation reaction with Thio-EPO is described in Example 3, 2.2.

### Alternatives:

In this reaction, all cross-linkers can be used, which exhibit a hydrazide- and a maleimide function, separated by a spacer. Further examples for molecules of that group, available from Perbio Science, Deutschland GmbH, Bonn, Germany, are shown in table 2; marked with an "A". Furthermore, another group of cross-linkers exhibiting an activated disulfide function instead of a maleimide funcion could also be used.
¹Manger, Wong, Rademacher, Dwek, 1992, Biochemistry, 31, 10733-10740; Manger, Rademacher, Dwek, 1992, Biochemistry, 31, 10724-10732

### 1.2 Halogenacetamide-derivatives of HES glycosylamines

### a) Glycosylamine-formation¹

A 1 mg sample of HES12KD was dissolved in 3 mL of saturated ammonium bicarbonate. Additional solid ammonium bicarbonate was then added to maintain saturation of the solution during incubation for 120 h at 30°C. The Amino-HES 12KD C was desalted by direct lyophilization of the reaction mixture.

### b) Acylation of the glycosylamine C with chloroacetic acid anhydride

A 1 mg sample of Amino-HES 12KD C was dissolved in 1 mL of 1 M sodium bicarbonate and cooled on ice. To this was added a crystal of solid chloroacetic acid anhydride (∼5 mg), and the reaction mixture was allowed to warm to room temperature. The pH was monitored and additional base was added if the pH dropped below 7.0. After two hours at room temperature a second aliquot of base and anhydride was added. After six hours the product Chloroacetamide-HES **D1** (X = Cl) was desalted by passage over a mixed bed Amberlite MB-3(H)(OH) ion exchange resins.
²*Black, Kiss, Tull, Withers, 1993,Carbohydr. Res., 250, 195*

### c) Acylation of the glycosylamine with bromoacetic anhydride²

Bromoacetic anhydride was prepared as described by Thomas.³ A 1 mg sample of amino-HES 12KD C was dissolved in 0.1 mL of dry DMF and cooled on ice and 5 mg bromoacetic anhydride was added. The reaction mixture was brought slowly to room temperature and the solution was stirred for 3 h. The reaction mixture was added to 1 mL of a 1:1 mixture of ethanol and acetone with -20 °C. The precipitate was collected by centrifugation, redissolved in 0.1 mL DMF and again precipitated as described. The Bromoacetamide-HES **D2** (X = Br) was obtained by centrifugation and drying in vaccuo. The conjugation reaction with Thio-EPO is described in Example 3, 1.2.
³Thomas, 1977, *Methodes Enzymol., 46*, 362

### d) The corresponding Iodo-derivative D3 (X = I) was synthesised as described for D2. Instead bromoacetic anhydride N-succinimidyl iodoacetate was used and all steps were performed in the dark.

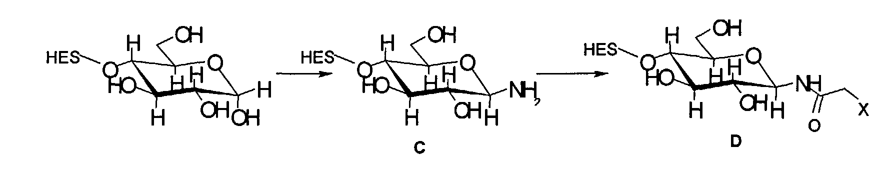

### Alternatives:

For acylation of amino groups, other activated forms of halogen acidic acids can be used, e.g.
- -bromides or -chlorides
- esters, e.g. N-hydroxysuccinimide ester, esters with substituted phenoles (p-nitrophenole, pentafluorophenole, trichlorophenole etc)

Furthermore, all cross-linkers having an amino reactive group and a halogen acetyl function, separated by a spacer, could be used. An example thereof is SBAP. This molecule and others are available from Perbio Science Deutsch- land GmbH, Bonn, Germany. They are marked in table 2 with an "D". For the use as cross-linkers for the ligation of amino-HES with thio-EPO without isolation of the halogenacetamid-HES derivatives see remarks in example 3, 1.2.
⁴S. Frie, Diplomarbeit, Fachhochschule Hamburg, 1998

### 1.3 Halogenacetamide-derivatives of Amino-HES E¹

### a) Reaction of 1,4-diaminobutane with Oxo-HES 12KD to amino-HES 12KD E⁴

1.44 g (0.12 mmol) of Oxo-HES 12KD were dissolved in 3 mL dry dimethyl sulfoxide (DMSO) and were added dropwise under nitrogen to a mixture of 1.51 mL (15 mmol) 1,4-diaminobutane in 15 mL DMSO. After stirring for 19 h at 40°C the reaction mixture was added to 160 mL of a 1:1 mixture of ethanol and acetone. The precipitate Amino-HES 12KD **E** was collected by centrifugation, redissolved in 40 mL of water an dialysed for 4 days against water (SnakeSkin dialysis tubing, 3.5 KD cut off, Perbio Science Deutschland GmbH, Bonn, Germany) and lyophilized.

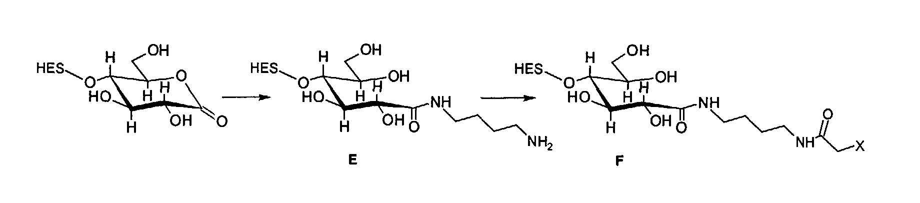

### b) Chloroacetamide-HES12KD F1 was prepared as described for Chloroacet-amide-HES12KD D1 in 1.3 above.

### c) Bromoacetamide-HES 12KD F2 (X = Br) was prepared as described for Bromoacetamide-HES12KD D2 in 1.3 above. The conjugation reaction with Thio-EPO is described in Example 3, 1.2.

### d) The corresponding Iodo-derivative F3 (X = I) was not isolated before its reaction with Thio-EPO. The experiment is described in Example 3, 1.1.

### Alternatives:

See 1.2 above

### 2. CHO-Reactive HES

### 2.1 Hydrazide-HES

### a) Reaction of hydrazine with Oxo-HES 12KD

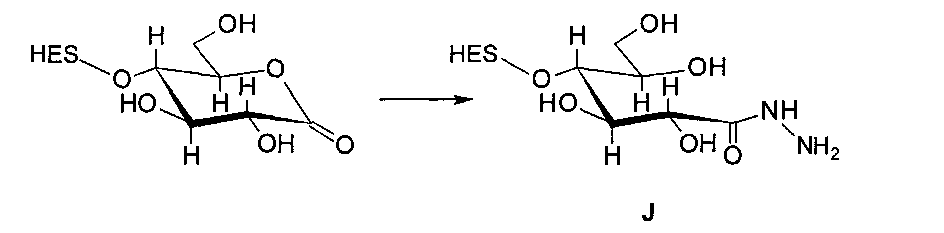

1,44 g (0.12 mmol) of Oxo-HES12KD were dissolved in 3 mL absolute dimethyl sulfoxide (DMSO) and were added dropwise under nitrogen to a mixture of 0.47 mL (15 mmol) hydrazine in 15 mL DMSO. After stirring for 19 h at 40°C the reaction mixture was added to 160 mL of a 1:1 mixture of ethanol and acetone. The precipitated product **J** was collected by centrifugation, redissolved in 40 mL of water and dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 4, 2.2.

### b) Reaction of adipic dihydrazide with Oxo-HES 12KD

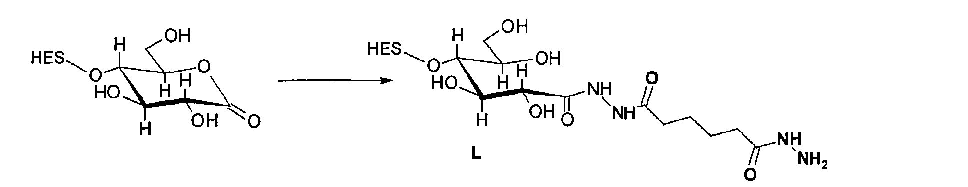

1.74 g (15 mmol) adepic dihydrazide were dissolved in 20 mL absolute dimethyl sulfoxide (DMSO) at 65°C and 1,44 g (0,12 mmol) of Oxo-HES12KD, dissolved in 3 mL absolute DMSO were added dropwise under nitrogen. After stirring for 68 h at 60°C the reaction mixture was added to 200 mL of water The solution containing **L** was dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 4, 2.2.

### Alternatives:

Furthermore, derivatives can be used, wherein 2 hydrazid groups are separated by any spacer.

### 3. Further Amino-HES12KD derivatives I and H¹

Ammonolysis of **D** or **F** was performed separately by dissolving a 1 mg sample of each halogeneacetamide in 0.1 mL of saturated ammonium carbonate. Additional solid ammonium carbonate was then added to maintain saturation of the solution during incubation of 120 h at 30°C. The reaction mixture was added to 1 mL of a 1:1 mixture of ethanol and acetone with ―20 °C. The precipitate was collected by centrifugation, redissolved in 0.05 mL water and again precipitated as described. The product aminoHES **H** or **I** was obtained by centrifugation and drying in vaccuo. The conjugation reaction with oxidised Glyco-EPO is described in Example 4, 4.1.

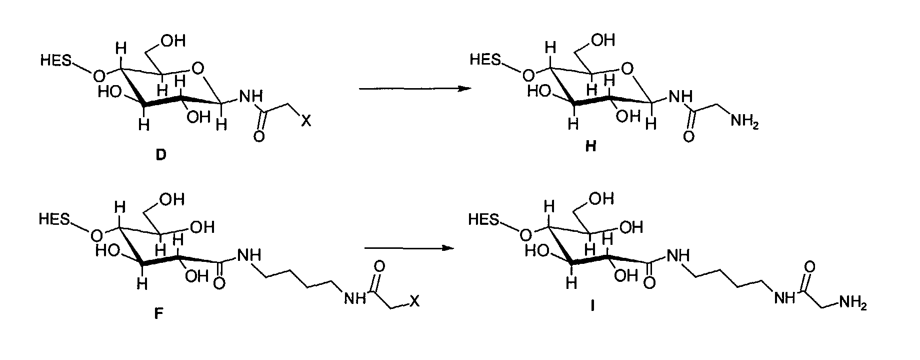

### 4. Hydroxylamine-modified HES12KD K

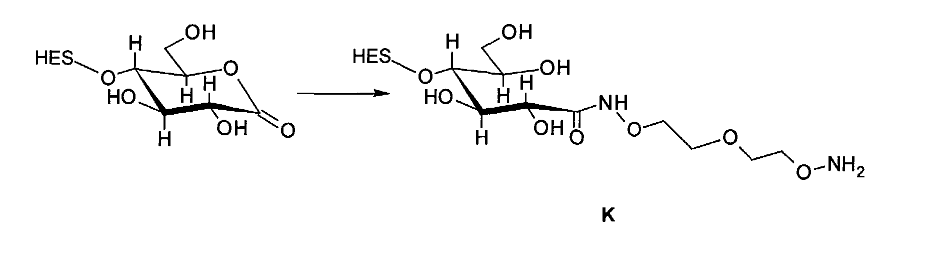

O-[2-(2-aminooxy-ethoxy)-ethyl]-hydroxylamine was synthesized as described by Boturyn et al in 2 steps from commercially available materials.⁵ 1,44 g (0.12 mmol) of Oxo-HES 12KD were dissolved in 3 mL absolute dimethyl sulfoxide (DMSO) and were added dropwise under nitrogen to a mixture of 2.04 g (15 mmol) O-[2-(2-aminooxy-ethoxy)-ethyl]-hydroxylamine in 15 mL DMSO. After stirring for 48 h at 65°C the reaction mixture was added to 160 mL of a 1:1 mixture of ethanol and acetone. The precipitated product **K** was collected by centrifugation, redissolved in 40 mL of water and dialysed for 4 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 4, 3.1.
⁵Boturyn, Boudali, Constant, Defrancq, Lhomme, 1997, *Tetrahedron, 53*, 5485

### Alternatives:

Furthermore, derivatives could be used, wherein the two hydroxylamine groups are separated by any spacer.

### 5. Thio-HES12KD

### 5.1 Addition to Oxo-HES12KD

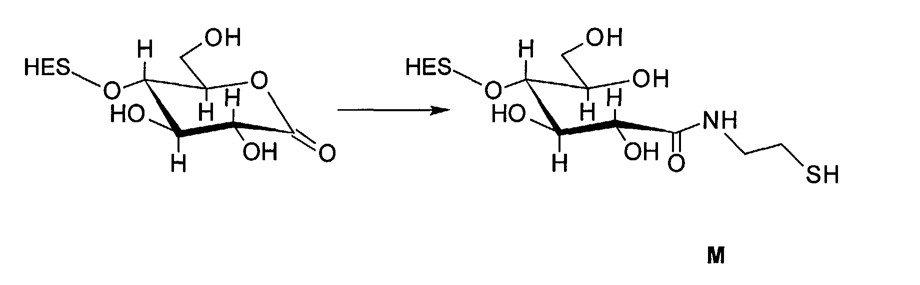

1,44 g (0.12 mmol) of Oxo-HES12KD were dissolved in 3 mL absolute dimethyl sulfoxide (DMSO) and were added to a mixture of 1.16 g (15 mmol) cysteamine in 15 mL DMSO under nitrogen dropwise. After stirring for 24 h at 40°C the reaction mixture was added to 160 mL of a 1:1 mixture of ethanol and acetone. The precipitated product **M** was collected by centrifugation, redissolved in 40 mL of water and dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 4, 2.1.

### Alternatives:

Derivatives could be used, wherein the amino group and the thio-function are separated by any spacer. Furthermore, the amino group in the derivatives could be replaced by a hydrazine, a hydrazid or a hydroxylamine. The thio-function could be protected in the form of e.g. a disulfide or a trityl-derivative. However, in this case, a further deprotection step must be preformed before the conjugation, which would release a component being analogous to **M**.

### 5.2 Modifikation of Amino-HES12KD E, H or I

### a) Modification with SATA/SATP

1,44 g (0.12 mmol) of Amino-HES12KD **E, H** or **I** were dissolved in 3 mL absolute dimethyl sulfoxide (DMSO) and were added to a mixture of 139 mg (0.6 mmol) SATA in 5 mL DMSO under nitrogen dropwise. After stirring for 24 h at room temperature the reaction mixture was added to 160 mL of a 1:1 mixture of ethanol and acetone. The precipitated product **N** was collected by centrifugation, redissolved in 40 mL of water and dialysed for 2 days against water (SnakeSkin dialysis tubing, 3.5 KD cut off, Perbio Science Deutschland GmbH, Bonn, Germany) and lyophilized.

The deprotection was performed in a 50 mM sodium phosphate buffer, containing 25 mM EDTA and 0.5M hydroxylamine, pH7.5 for 2 hours at room temperature and the product **O** was purified by dialysis against a 0.1 M sodium acetate buffer pH 5.5, containing 1 mM EDTA. The deprotection reaction was performed immediately before the conjugation reaction which is described in Example 4, 2.1.

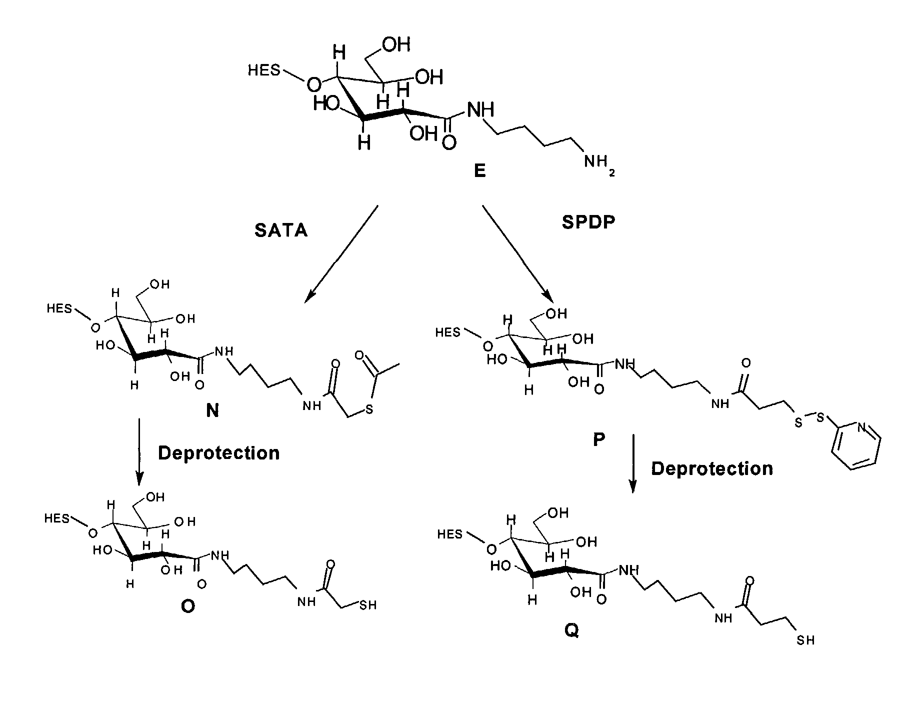

### b) Modification with SPDP

1,44 g (0.12 mmol) of Amino-HES12KD **E, H** or **I** were dissolved in 3 mL absolute dimethyl sulfoxide (DMSO) and were dropwise added to a mixture of 187 mg (0.6 mmol) SPDP in 5 mL DMSO under nitrogen. After stirring for 24 h at room temperature the reaction mixture was added to 160 mL of a 1:1 mixture of ethanol and acetone. The precipitated product **P** was collected by centrifugation, redissolved in 40 mL of water and dialysed for 2 days against water (SnakeSkin dialysis tubing, 3.5 KD cut off, Perbio Science Deutschland GmbH, Bonn, Germany) and lyophilized.

The deprotection was performed in a solution of 12 mg dithiothreitol (DTT) per 0.5 mL 100 mM sodiumacetate buffer, containing 100 mM sodium chloride at pH 4.5 for 30 min at room temperature and the product **Q** was purified by dialysis against a 0.1 M sodium acetate buffer pH 5.5, containing 1 mM EDTA. The deprotection reaction was performed immediately before the conjugation reaction which is described in Example 4, 2.1.

### Alternatives:

For the conversion of amino- to thiol-groups, either in free form or protected, several reagants are available. After the modification, the products could be isolated. Alternatively, as accepted for the use of cross-linkers, they could be directly used for the conjugation reaction, preferably after a purification step. For the isolation and storage of thio-HES derivatives, the synthesis of thio-HES derivatives in a protected form may be useful. For this, all derivatives being analogous to SATA could be used, which have an active ester-function and a thioester-function, separated by any spacer. SATP, being a further member of this group, is found in table 2, marked with an "H". The derivatives being analogous to SPDP could have an acitve ester-function and a disulfide-function, separated by any spacer. Further members of these groups are found in table 2, marked with an "F". Further analogous derivatives could have an active ester-function and a thiol-function, protected as a trityl derivative, separated by any spacer.

### Example 3

### Conjugation reactions with Thio-EPO

### 1. Reaction of Thio-EPO with a halogenacetamide-modified SH-reactive HES

### 1.1 Example Protocol 1

Conjugation of ThioEPO to Amino-HES12KD **(E, H** or **I)** with a Cross-linker containing a NHS-active-ester and an iodoacetamide group, e.g. SIA.⁶
⁶Cumber, Forrester, Foxwell, Ross, Thorpe, 1985, *Methods Enzymol., 112*, 207

### Materials

A. Borate buffer. Composition was 50 mM sodium borate, pH 8.3, 5 mM EDTA.
B. PBS, phosphate buffered saline: 10 mM sodium phosphate, 150 mM NaCl, pH 7.4.
C. AminoHES12KD **E, H** or **I.** Prepared at 1 mg/mL in borate buffer.
D. Crosslinker stock solution: 14 mg SIA were dissolved in 1 mL DMSO
E. D-Salt^{TM} Dextran Desalting Columns, 2 x 5 mL bed volume (Perbio Science Deutschland GmbH, Bonn, Germany)
F. Coomassie® Protein Assay Reagent (Perbio Science Deutschland GmbH, Bonn, Germany)
G. ThioEPO solution: 5 mg/mL of ThioEPO **1** in borate buffer.
H. Microconcentrator: Microcon YM-3 (amicon, Milipore GmbH, Eschborn, Germany)

### Method

100 µL SIA solution was added to 400 µL of the aminoHES 12KD **E** solution and was allowed to react with agitation for 0.5 hours at room temperature. The excess crosslinker was removed by centrifuging the sample at 14000 x g for 60 minutes using a microconcentrator. After centrifuging the sample was brought up to its original volume in borate buffer and this process was repeated two more times. The residual solution was added to 1 mL of ThioEPO solution and the reaction mixture was incubated for 16 hour at room temperature. Reactivity of the excess iodoacetamide was quenched at the end of the incubation period by the addition of cysteine to a final concentration of 10 mM. The reaction mixture was applied to a desalting column equilibrated with PBS buffer and the protein content of the fractions were monitored with a Coomassie protein assay reagent. All fractions containing the protein conjugate were pooled and the the conjugate was obtained by lyophylisation after dialysis against water over night.

### Alternatives:

In this reaction, all cross-linkers could be used, which have a succinimide- or a sulfosuccinimide function and a iodoacetamide function separated by a spacer. Further examples are found in table 2. They are marked with a "C" and are avialable from Perbio Science Deutschland GmbH, Bonn, Germany.

### 1.2 Example Protocol 2

Conjugation of ThioEPO **1** to SH reactiveHES12KD bromoacetamide **D2, F2** or iodoacetamide **D3.**⁷
⁷de Valasco, Merkus, Anderton, Verheul, Lizzio, Van der Zee, van Eden, Hoffmann, Verhoef, Snippe, 1995, *Infect. Immun., 63*, 961

### Materials

A. Phosphate buffer. Composition was 100 mM sodium phosphate, pH 6.1, 5 mM EDTA.
B. PBS, phosphate buffered saline: 10 mM sodium phosphate, 150 mM NaCl, pH 7.4.
C. SH reactiveHES12KD bromoacetamide **D2**. Prepared at 10 mg/mL in phosphate buffer.
D. D-Salt^{TM} Dextran Desalting Columns, 2 x 5 mL bed volume (Perbio Science Deutschland GmbH, Bonn, Germany)
E. Coomassie® Protein Assay Reagent (Perbio Science Deutschland GmbH, Bonn, Germany)
F. ThioEPO solution: 5 mg/mL of ThioEPO **1** in phosphate buffer.

### Method

1 mL SH reactiveHES 12KD bromoacetamide **D2** solution and 1 mL of Thio-EPO solution were combined and the reaction mixture was incubated for 48 hours at room temperature. Reactivity of the excess bromoacetamide was quenched at the end of the incubation period by the addition of cysteine to a final concentration of 10 mM. The reaction mixture was applied to a desalting column, equilibrated with PBS buffer. The protein content of the fractions were monitored with a Coomassie protein assay reagent, all fractions containing the protein conjugate were pooled and the the conjugate was obtained by lyophylisation after dialysis against water over night.

### Alternatives:

Instead of the isolation of the SH reactive HES12KD-bromoacetamid **D2**, amino HES12KD **(E, H, I)** could be linked with a cross-linker via a succinimide- and a bromoacetamid function (see 1.1 above). SBAP is a member of this group of cross-linkers and is found in table 2, marked with a "D".

### 2. Reaction of Thio-EPO with a maleimide-modified SH-reactive HES

### 2.1 Example Protocol 3

Conjugation of ThioEPO to HES12KD with a cross-linker containing a hydrazide and a maleimide funktional group, e.g. M₂C₂H.

### Materials

A. M₂C₂H stock: 10 mg/mLM2C2H in DMSO, prepared fresh
B. HES 12KD: 10 mg/mL in 0.1 M sodium acetate buffer, pH 5.5
C. ThioEPO solution: 5 mg/mL of ThioEPO in phosphate/NaCI-buffer
D. Phosphate/NaCI: 0.1 M sodium phosphate, 50 mM NaCI, pH 7.0
E. Microconcentrator: Microcon YM-3 (amicon, Milipore GmbH, Eschborn, Germany)
F. Gel filtration column: for example, Sephadex® G-200 (1.5 x 45 cm)
G. Coomassie® Protein Assay Reagent (Perbio Science Deutschland GmbH, Bonn, Germany)
H. PBS, phosphate buffered saline: 10 mM sodium phosphate, 150 mM NaCl, pH 7.4.

### Method

M₂C₂H solution was added to 400 µL of the HES 12KD solution to a final concentration of 1 mM and was allowed to react with agitation for 2 hours at room temperature. The excess cross-linker was removed by centrifuging the sample at 14000 x g for 60 minutes using a microconcentrator. After centrifuging the sample was brought up to its original volume in phosphate/NaCI buffer and this process was repeated two more times. To the M₂C₂H-modified HES 12KD 0.5 mL of ThioEPO solution was added and the reaction mixture was incubated for 2 hours at room temperature. Reactivity of the excess maleimides was quenched at the end of the incubation period by the addition of cysteine to a final concentration of 10 mM. The reaction mixture was applied to Sephadex® G-200 (1.5 x 45 cm) equilibrated with PBS buffer and 1 mL fractions were collected. The protein content of the fractions were monitored with a Coomassie protein assay reagent. All fractions containing the protein conjugate were pooled and the the conjugate was obtained by lyophylisation after dialysis against water over night.

### Procedural Notes

The hydrazone adduct is slightly less stable at extremes of pH. For applications that may involve treatment at low pH, we reduced the hydrazone by treatment with 30 mM sodium cyanoborohydride in PBS buffer to a hydrazine. For most applications, this extra step is unnecessary.

### 2.2 Example Protocol 4

Conjugation of ThioEPO to Maleimido-HES12KD **B**.

### Materials

A. Maleimido-HES 12KD **B**: 10 mg/mL in 0.1 M sodium acetate buffer, pH 5.5
B. ThioEPO solution: 5 mg/mL of ThioEPO in phosphate/NaCI-buffer
C. Phosphate/NaCI: 0.1 M sodium phosphate, 50 mM NaCI, pH 7.0
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline: 10 mM sodium phosphate, 150 mM NaCl, pH 7.4.

### Method

1 mL SH-reactive-HES12KD **B** solution and 1 mL of ThioEPO **1** solution were combined and the reaction mixture was incubated for 2 hours at room temperature. Reactivity of the excess maleimides was quenched at the end of the incubation period by the addition of cysteine to a final concentration of 10 mM. The reaction mixture was applied to Sephadex® G-200 (1.5 x 45 cm) equilibrated with PBS buffer and 1 mL fractions were collected. The protein content of the fractions were monitored with a Coomassie protein assay reagent. All fractions containing the protein conjugate were pooled and the the conjugate was obtained by lyophylisation after dialysis against water over night.

### 2.3 Example Protocol 12

Conjugation of ThioEPO to aminoHES12KD (**E**, **H**, **I**) with a Cross-linker containing a NHS-active-ester and a maleimide group, e.g. SMCC

### Materials

A: Microconcentrator: Microcon YM-10 (amicon, Milipore GmbH, Eschborn, Germany).
B. PBS, phosphate buffered saline: 10 mM sodium phosphate, 150 mM NaCl, pH 7.4.
C. AminoHES12KD **E**, **H** or **I.** Prepared at 10 mg/mL in PBS buffer.
D. SMCC solution: 1 mg SMCC were dissolved in 50 µL DMSO
E. D-Salt™ Dextran Desalting Columns, 2 x 5 mL bed volume (Perbio Science Deutschland GmbH, Bonn, Germany)
F. Coomassie® Protein Assay Reagent (Perbio Science Deutschland GmbH, Bonn, Germany)
G. ThioEPO **1** solution: 5 mg/mL of ThioEPO **1** in PBS buffer.

### Method

To 50 µL SMCC solution 400 µL of the aminoHES12KD **E** solution was added and the reaction mixture was allowed to react with agitation for 80 min at room temperature and for 10 min at 46°C. The excess crosslinker was removed by centrifugation of the reaction mixture through a microconcentrator at 14000 x g for 60 min. The volume was brought up to 450 µL with PBS buffer and the process was repeated two more times. After the last centrifugation, the residual solution was brought up to 450 µL with PBS and was added to 1 mL of ThioEPO solution and the reaction mixture was incubated for 16 hours at room temperature. Reactivity of the excess maleimide was quenched at the end of the incubation period by the addition of cysteine to a final concentration of 10 mM. The reaction mixture was applied to a desalting column equilibrated with PBS buffer. The protein content of the fractions were monitored with a Coomassie protein assay reagent, all fractions containing the protein conjugate were pooled and the conjugate was obtained by lyophylisation after dialysis against water over night.

### Alternatives:

In this reaction, all cross-linkers could be used which have a succinimide- or a sulfosuccinimide function and a maleimide-function, separated by a spacer. Further examples for this group of molecules, available from Perbio Science Deutschland GmbH, Bonn, Germany, are found in table, 2, marked with an "E". There is a further group of cross-linkers, which have instead of a maleimide function an activated disulfide function. These cross-linkers could also be used for the conjugation. However, the disulfide bond of the conjugate is cleavable under reductive conditions. Members of this group are marked in table 2 with a "F". A third group of cross-linkers uses instead of a maleimide function a vinylsulfon function as a SH-reactive group. A member of this group "SVSB" is marked in table 2 with a "G".

### Example 4

### Conjugation reactions with oxidized EPO

### 1. Oxidation of Glyco-EPO

### 1.1 Oxidation of Glyco-EPO with sodium meta-periodate: Example Protocol 5

### Materials

A. Glyco-EPO solution: 10 mg/mL of Glyco-EPO in acetate buffer
B. Sodium meta-periodate solution: 10 mM or 100 mM sodium periodate in acetate buffer, prepared fresh. Keep in dark. Using these solutions, the final concentration of sodium periodate in the oxidation mixture is 1 mM or 10 mM, respectively.
C. acetate buffer: 0.1 M sodium acetate buffer, pH 5.5
D. Glycerol
E. Microconcentrator: Microcon YM-3 (amicon, Milipore GmbH, Eschbom, Germany)

### Method

All steps were performed in the dark.

To 1 mL of cold Glyco-EPO solution 0.1 mL of cold sodium meta-periodate solution were added and the the oxidation reaction was allowed to proceed for 1 hour in the dark. If the Glyco-EPO to be oxidized contained sialic acid residues, then the oxidation conditions were 1 mM sodium periodate, 0°C. Otherwise, 10 mM sodium periodate at room temperature was used. To stop the oxidation glycerol was added to a final concentration of 15 mM and incubated for 5 minutes at 0°C. The excess reagents and by-products were remove by centrifuging of the product at 14000 x g for 60 minutes using a microconcentrator. After centrifuging, sample was brought up to its original volume in the buffer used in the next modification step, e.g. in the acetate buffer. This process was repeated two more times.

### 1.2 Enzymatic oxidation of Glyco-EPO: Example Protocol 6

The enzymatic oxidation of EPO is described elsewhere (Chamow et al., 1992, J. Biol. Chem., 267, 15916-15922).

### 2. Conjugation with Hydrazine/Hydrazide-Derivatives

### 2.1 Example Protocol 7

Conjugation of oxidised Glyco-EPO to Thio-HES12KD **M**, **O** or **Q** with a Cross-linker containing a hydrazide and a maleimide functional group, e.g.M₂C₂H (Perbio Science, Deutschland GmbH, Bonn, Germany).

### Materials

A. M₂C₂H stock: 10 mg/mL M₂C₂H in DMSO, prepared fresh
B. Oxidised Glyco-EPO solution from 6.1.1: 5 mg/mL of Glyco-EPO in acetate buffer
C. Thio-HES12KD **M, O** or **Q:** 10 mg/mL in phosphate/NaCl buffer
D. Acetate buffer: 0.1 M sodium acetate buffer, pH 5.5
E. Phosphate/NaCI: 0.1 M sodium phosphate, 50 mM NaCI, pH 7.0
F. Microconcentrator: Microcon YM-3 (amicon, Milipore GmbH, Eschborn, Germany)
G. Gel filtration column: for example, Sephadex® G-200 (1.5 x 45 cm)
H. Coomassie® Protein Assay Reagent (Perbio Science Deutschland GmbH, Bonn, Germany)
I. PBS, phosphate buffered saline:10 mM sodium phosphate, 150 mM NaCl, pH 7.4

### Method

M₂C₂H stock solution was added to 1 mL of oxidized Glyco-EPO to a final concentration of 1 mM and was allowed to react with agitation for 2 hours at room temperature. The excess crosslinker was removed by centrifuging the sample at 14000 x g for 60 minutes using a microconcentrator. After centrifuging the sample was brought up to its original volume in phosphate/NaCI buffer and this process was repeated two more times. To the M₂C₂H-modified Glyco-EPO 1 mL of Thio-HES12KD M, O or Q solution was added and the reaction mixture was incubated for 16 hours at room temperature. Reactivity of the excess maleimides was quenched at the end of the incubation period by the addition of cysteine. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the conjugate was obtained by lyophylisation after dialysis against water over night.

### Procedural Notes

The hydrazone adduct is slightly less stable at extremes of pH. For applications that may involve treatment at low pH, we reduced the hydrazone by treatment with 30 mM sodium cyanoborohydride in PBS buffer to a hydrazine. For most applications, this extra step was unnecessary.

### 2.2 Example Protocol 8

Direct conjugation of oxidised Glyco-EPO to Hydrazido-HES 12KD **L** or **J**.

### Materials

A. Oxidised Glyco-EPO solution from 6.1.1: 5 mg/mL of Glyco-EPO in acetate buffer
B. Hydrazido-HES 12KD **L** or **J**: 10 mg/mL in acetate buffer
C. Acetate buffer: 0.1 M sodium acetate buffer, pH 5.5
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline:10 mM sodium phosphate, 150 mM NaCl, pH 7.4

### Method

1 mL of Hydrazido-HES12KD L or J solution and 1 mL of oxidized Glyco-EPO solution were combined and the reaction mixture was allowed to react with agitation for 16 hours at room temperature. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the the conjugate was obtained by lyophylisation after dialysis against water over night. The result of the conjugation is shown in Figure 1. The observed molecular shift demonstrates that the conjugation was successful. The smear results from the heterogenity of HES. Figure 2 demonstrates that HES is conjugated to a carbohydrate moiety of a carbohydrate side chain.

### Procedural Notes

The hydrazone adduct is slightly less stable at extremes of pH. For applications that may involve treatment at low pH, we reduced the hydrazone by treatment with 30 mM sodium cyanoborohydride in PBS buffer to a hydrazine. For most applications, this extra step was unnecessary.
⁸Rose, 1994, *Am. Chem. Soc., 116,* 30

### 3. Conjugation with Hydroxylamine-Derivatives⁸

### 3.1 Example Protocol 9

Conjugation of oxidized Glyco-EPO to Hydroxylamino-HES12KD **K**

### Materials

A. Oxidised Glyco-EPO solution from 6.1.1: 5 mg/mL of Glyco-EPO in acetate buffer
B. Hydroxylamino-HES12KD **K**: 10 mg/mL in acetate buffer
C. Acetate buffer: 0.1 M sodium acetate buffer, pH 5.5
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline: 10 mM sodium phosphate, 150 mM NaCl, pH 7.4

### Method

1 mL of Hydroxylamino-HES12KD K solution and 1 mL of oxidized Glyco-EPO solution were combined and the reaction mixture was allowed to react with agitation for 16 hours at room temperature. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the conjugate was obtained by lyophylisation after dialysis against water over night. The result of the conjugation is shown in Figure 1. The observed molecular shift in lane 2 demonstrates that the conjugation was successful. The smear results from the heterogenity of HES. Figure 2 demonstrates that HES is conjugated to a carbohydrate moiety of a carbohydrate side chain.

### Example 5

### Characterisation of galactose oxidase treated EPO N-glycans

Recombinant EPO or partially desialylated EPO forms (generated by limited mild acid hydroysis) were incubated with galactose oxidase in the presence of catalase at 37°C from 30 min - 4 hours at 37°C in 0.05 M Na-phosphate buffer pH 7.0. Progress of the reaction was monitored by removal of 50 µg aliquots of the EPO and subsequent treatment of the protein with polypeptide N-glycanase.

Liberated N-linked oligosaccharides (monitored by SDS-PAGE detection of the de-N-glycosylated polypeptide) were subjected to HPAEC-PAD mapping as described ( Grabenhorst et al., 1999, Nimtz et al., 1993/1994; Schlenke et al., 1999) before and after removal of sialic acids. Quantitation of oxidised galactose residues in individual EPO oligosaccharides was performed by the typical shift observed in HPAEC-PAD and was also verified by MALDI/TOF MS of the oligosaccharide mixtures.

### Example 6

### Characterisation of HAS modified EPO

Separation of HAS modified EPO forms from nonreacted EPO and HAS-precursor molecules was achieved by gel filtration using e.g. Ultrogel AcA 44 / 54 or similar gel filtration media. Alternatively, nonreacted HAS was removed by immuno affinity isolation of EPO on a 4 mL column containing a monoclonal antibody coupled to Affigel (BioRad) and subsequent separation of unmodified EPO by gel filtration (e.g. using a matrix enabling the separation of globular proteins of a relative molecular mass between 20 kDa and 200 kDa ).

HAS modified EPOs were identified by SDS-PAGE analysis (using 12.5 or 10% acrylamide gels) through detection of their higher molecular weight compared to unmodified EPO upon staining of gels with Coomassie Brillant Blue. The higher molecular weight of HAS modified EPO polypeptides was also identified by Western Blot analysis of samples using a polyclonal antibody raised against recombinant human EPO.

N-glycan modification of EPO forms was demonstrated by their successful removal from the EPO protein with polypeptide N-glycanase (recombinant N-glycosidase from Roche, Germany employing 25 units / mg EPO protein at 37°C for 16 hours); analysis by SDS-PAGE resulted in a typical shift of the EPO protein to a migration position of the N-glycosidase treated unmodified EPO of approximately 20 KDa.

Modification of the single desialylated and glacatose oxidase treated EPO O-glycan at Ser 126 was demonstrated by SDS-PAGE migration of the de-N-glycosylated product by detection of its migration position compared to nonreacted de-N-glycosylated EPO. If required, modified EPO was fractionated by RP-HPLC on a C8-phase before SDS-PAGE analysis. HAS O-glycan modification of EPO was also analysed by β-elimination of the O-glycan and detection of the de-O-glycosylated form of EPO in Western blots using a polyclonal antibody raised against recombinant human EPO.

### Example 7

### Quantitation of EPO and modified EPO forms

EPO forms where quantitated by UV measurements as described in Ph.Eur (2000, Erythropoietini solutio concentrata, 1316, 780-785) and compared to the international BRP reference EPO standard. Alternatively, EPO concentrations were determined by a RP-HPLC assay using a RP-C4-column and absorption at 254 nm employing 20, 40 , 80 and 120 µg of the BRP standard EPO reference preparation for calibration.

### Example 8

### In-vitro biological actvity of HES-modified recombinant human EPO:

Purified HES-modified EPO was tested for activity using the erythropoietin bioactivity assay as described by Krystal [Krystal, 1984, Exp. Heamatol., 11, 649-660].

Anemia was induced in NMRI mice by treatment with phenylhydrazine hydrochloride and spleen cells were collected and used as described in [Fibi et al., 1991, Blood, 77, 1203 ff.]. Dilutions of EPO were incubated with 3x10⁵ cells/well in 96-well microtiter plates. After 24 hours at 37° C in a humified atmosphere (5% CO₂) cells were labelled for 4 hours with 1 µCi of ³H-thymidine per well. Incorporated radioactivity was determined by liquid scintillation counting. The International reference EPO standard (BRP-standard) was used for comparison.

Alternatively, EPO bioactivity was measured by an in vitro assay using the EPO-sensitive cell line TF-1 (Kitamura et. al., [J. cell Phys., 140. 323-334]. Exponentially growing cells were washed free of growth factors and were incubated in the presence of serial dilutions of the EPO for further 48 hours. Proliferation of the cells was assessed by using the MTT reduction assay as described by Mosmann [Mosman, 1983, J.Immunol. Methods, 65, 55-63].

### Example 9

### In-vivo activity determination of EPO and HAS-modified EPO forms:

In vivo activity determinations were performed in normocythemic mice by measuring the increase of reticulocytes after 4 days after animals received the foreseen dose of EPO or modified EPO forms. Assays were performed using the BRP EPO standard which was calibrated against the WHO EPO standard in the polycythemic mouse assay. EPO samples were diluted in phosphate buffered saline containing 1 mg/ml of bovine serum albumin (Sigma).

0.5 ml of the EPO test solution in Dulbecco's buffered saline (corresponding to an EPO protein equivalent of a 100, 80, 40 or 20 IU/ml of the BRP standard EPO) were infected subcutaneously per animal. Blood samples were taken after 4 days after injection and reticulocytes were stained with acridine orange; quantitation of reticulocytes was performed by flow-cytometry by counting a total of 30,000 blood cells within 5 hours after the blood sample was taken (see Ph. Eur, 2000, Erythropoietini solutio concentrata, 1316, pages 780-785) and European Pharmacopoeia (1996/2000, attachment 2002).

### Example 10

### In-vivo half-life Determinations

Rabbits were injected intravenously with specified amounts of unmodified or HAS-modified EPO forms. Blood samples were obtained at specified times, and serum was prepared. Serum erythropoietin levels were determined by *in vitro* bioassay or by an EPO-specific commercial ELISA.

### Example 11

### In vivo pharmakokinetics

In mice: Each animal received 300 IU EPO/kg subcutaneously. Seven days after the post-treatment hematocrit of each animal was determined. A substantial increase in hematocrit was observed 9in all animals treated with modified EPO, an expected result in view o the relatively short half-life of untreated EPO. The mean change in hematocrit of the modified EPO-treated group was significantly different from that of the untreated EPO group and that of the control group.

In rabbits: Rabbits were treated with a single dose of unmodified or HAS-modified EPO corresponding to 200 or up to 800 ng/kg body weight. After 2, 6, 16, 24 and 48 hours blood samples were analyzed by using a commercial EPO-specific ELISA for determination of plasma concentrations. Mean plasma EPO concentrations were determined and the average initial half-lives (α-phase) and the terminal half-lives (β-phase) were calculated from the ELISA values as described: (Zettlmissl et al., 1989, J. Biol. Chem., 264, 21153-21159).

### Literature:

Sytkowski, Lunn, Risinger, and Davis, 1999, An Erythropoietin Fusion Protein Comprised of Identical Repeating Domains Exhibitis Enhanced Biological Properites, J. Biol. Chem., 274, 24773-24778.

### Example 12

### Assessment of the in vitro biological activity of HES-modified recombinant human IL-2

Modified IL2 was recovered by gelfiltration on Ultrogel AcA 54. Aliquots of corresponding fraction were sterile filtrated and IL2 bioactivity was determined by using the IL2 dependent murine CTLL-2 cell line [Gillis, Ferm, On, and Smith, 1978, J.Immunol., 120, 2027-2032]. Activity was related to the international reference IL2 standard preparation.

**Table 1**

| **Linker-type** | **Functional group 1: Reaction with polypeptide, especially EPO** | **Functional group 2: Reaction with HES** |
|---|---|---|
| A | Hydrazide (aldehyde-reactive) | Maleimido (SH-reactive |
| B | Hydrazide (aldeyde-reactive) | Pydridydithio (SH-reactive) |
| C | Iodoalkyl (SH-reactive) | N-succinimide ester (amine-reactive) |
| D | Bromoalkyl (SH-reactive) | N-succinimide ester (amine-reactive) |
| E | Maleimido (SH-reactive) | N-succinimide ester (amine-reactive) |
| F | Pydridyldithio (SH-reactive) | N-succinimide ester (amine-reactive) |
| G | Vinylsulfone (SH-reactive) | N-succinimide ester (amine-reactive) |

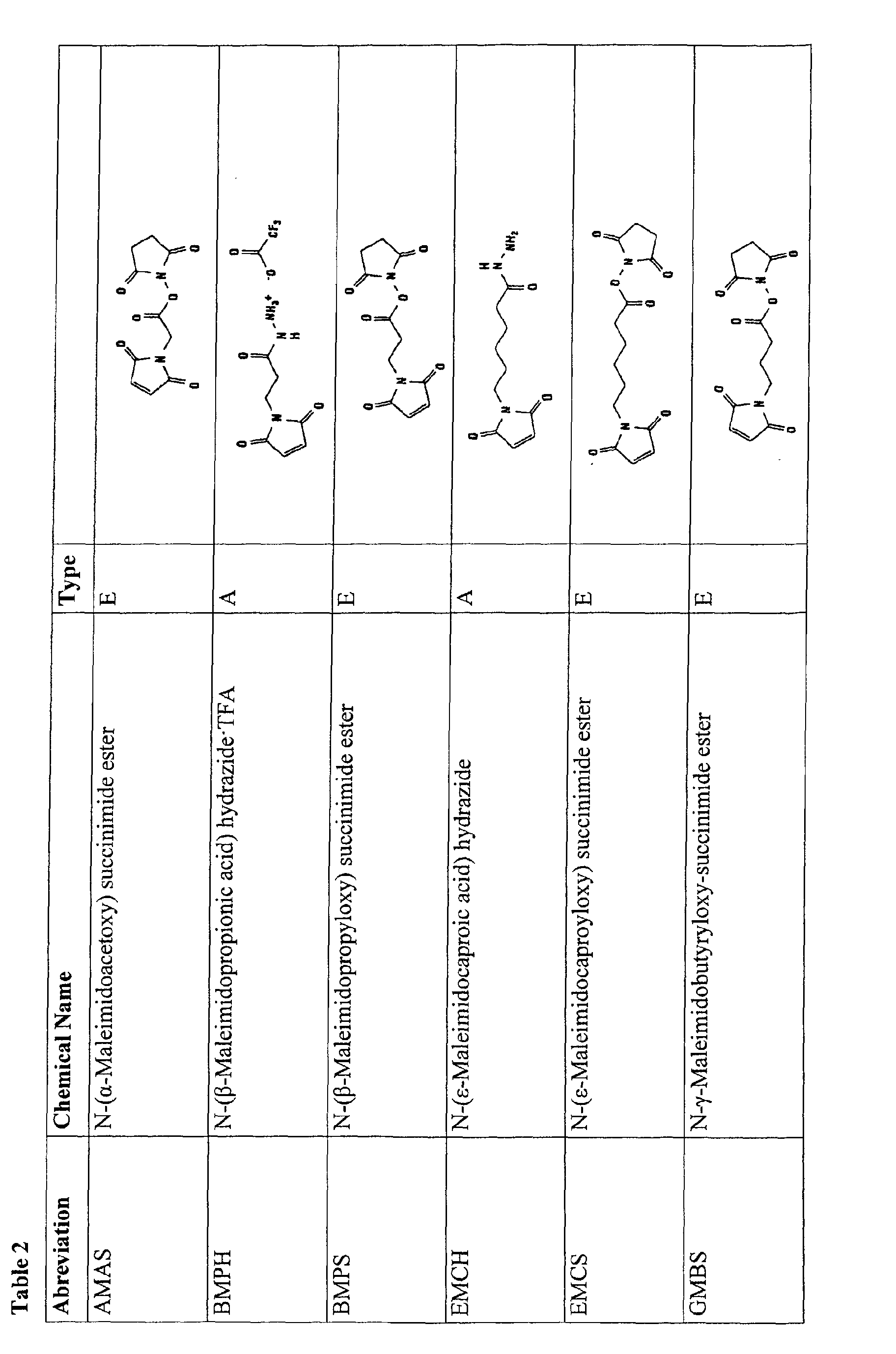

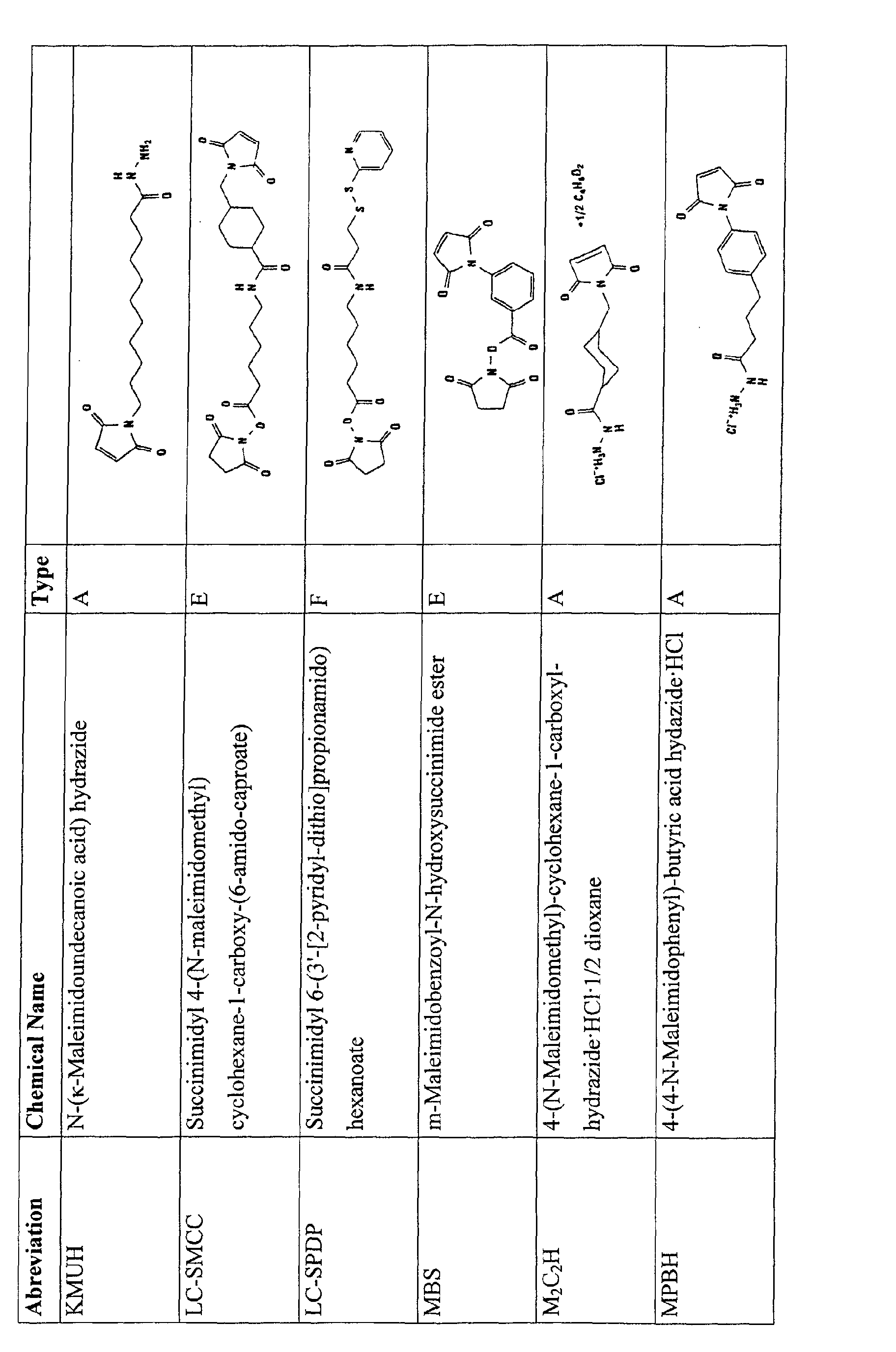

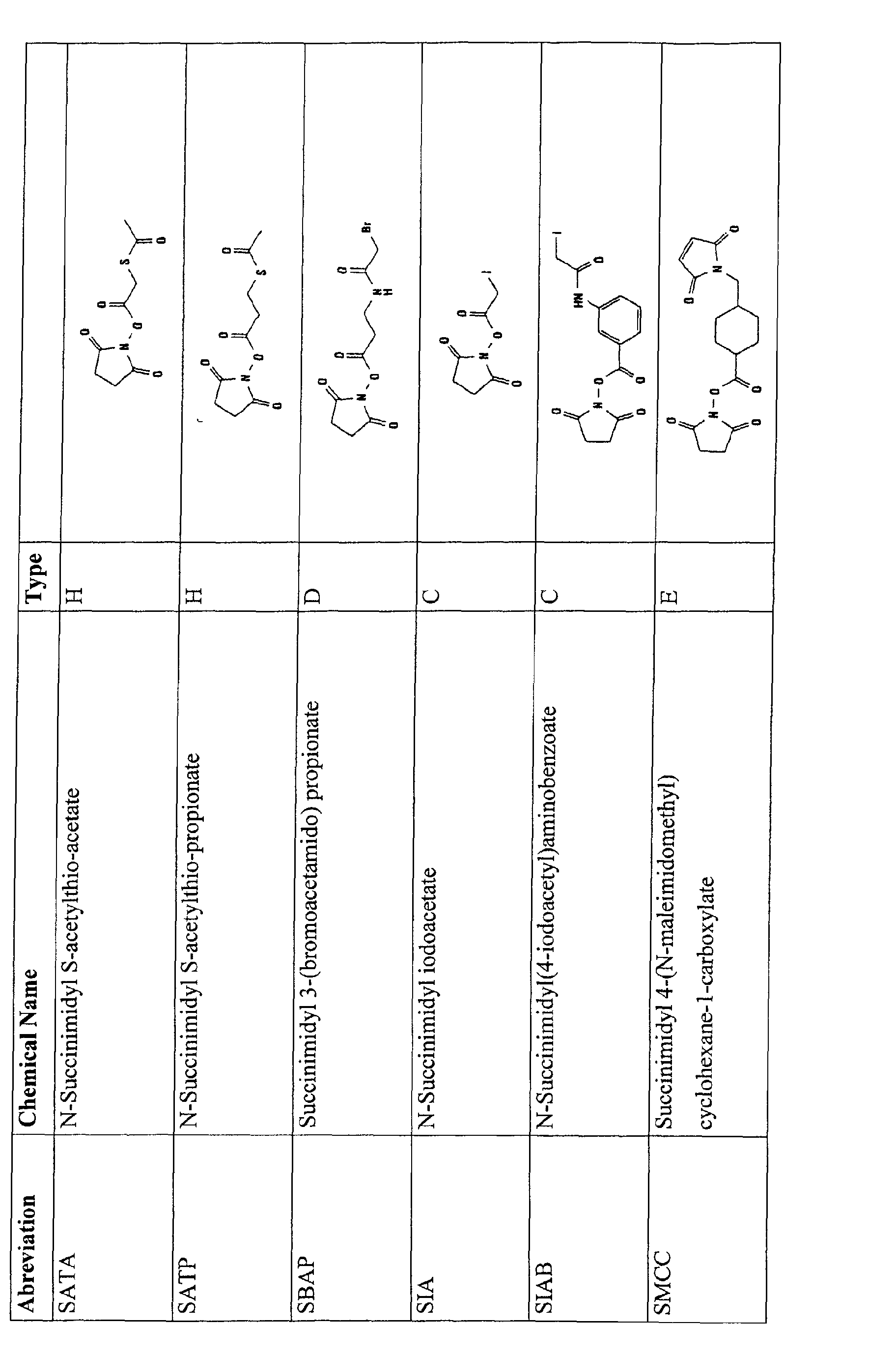

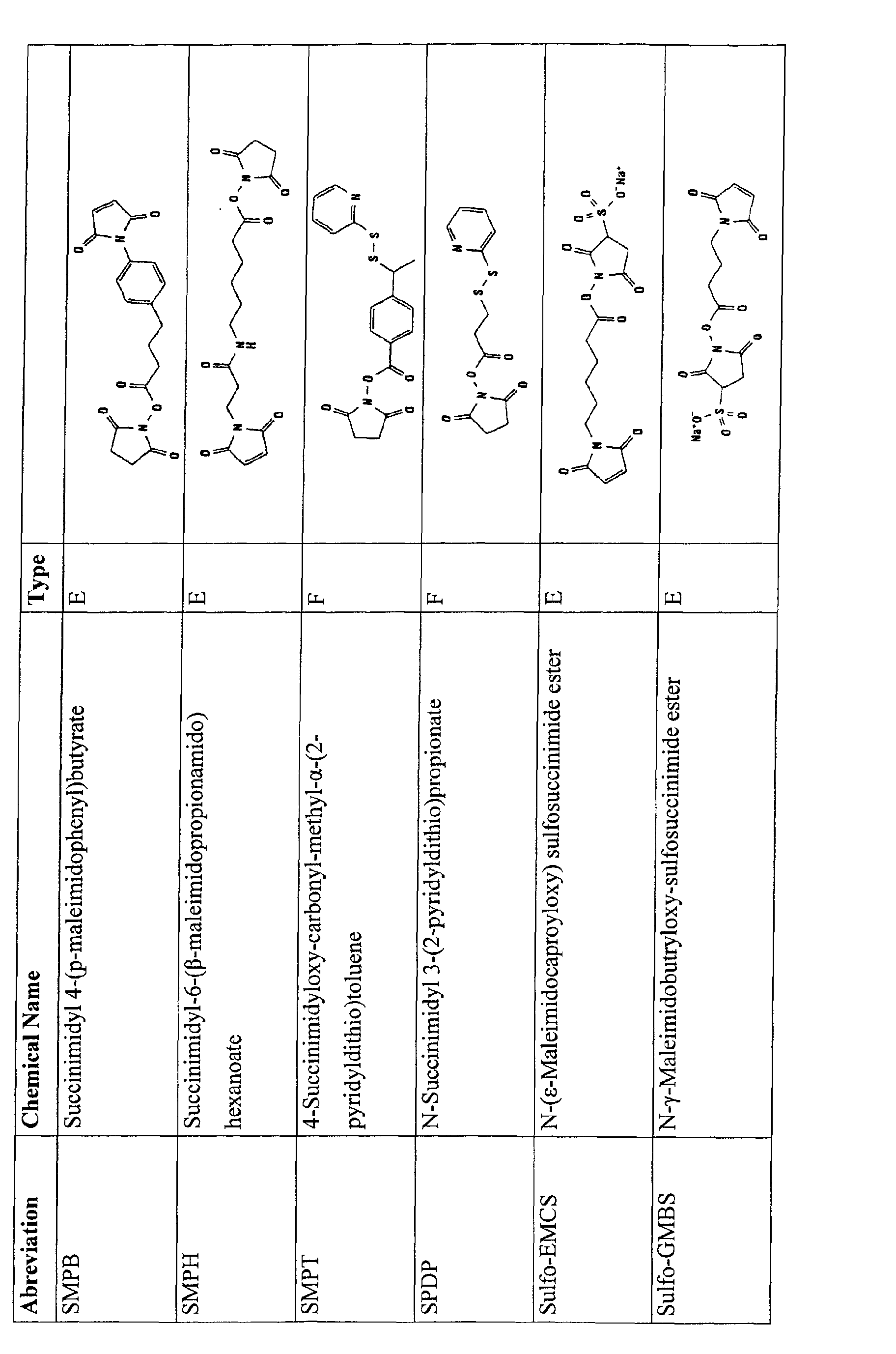

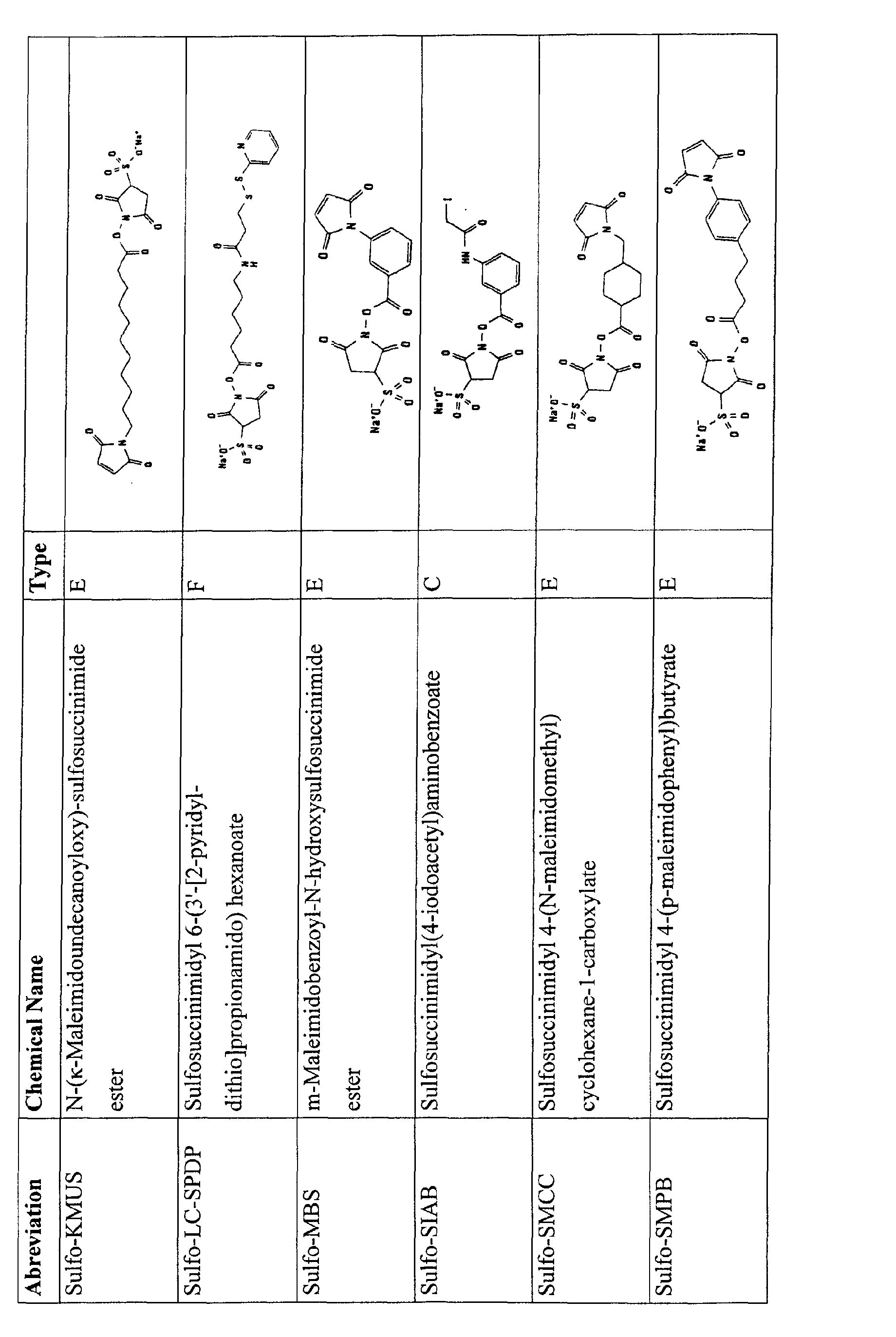

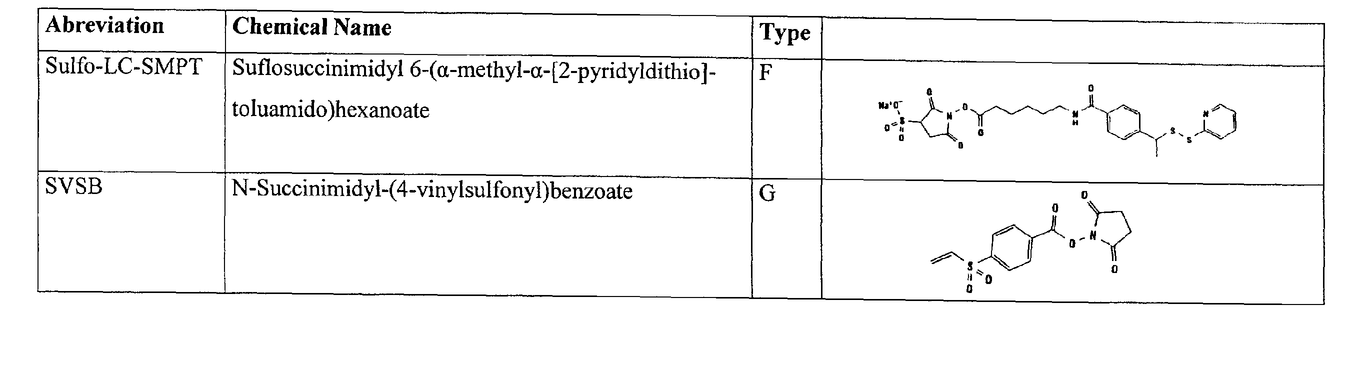

## Claims

1. A hydroxyalkylstarch (HAS)-erythropoietin (EPO)-conjugate (HAS-EPO), comprising one or more HAS molecules, wherein each HAS is conjugated to the EPO via
a) a carbohydrate moiety; or
b) a thioether.

2. The HAS-EPO of claim 1, wherein the EPO has the amino acid sequence of human EPO.

3. The HAS-EPO of any of claims 1 or 2, wherein the EPO comprises one or more carbohydrate side chains attached to the EPO via N- and/ or O-linked glycosylation.

4. The HAS-EPO of claim 3, wherein the carbohydrate side chains have been attached to the EPO during production in mammalian, especially human, insect or yeast cells.

5. The HAS-EPO of any of claims 1 to 4, wherein HAS is conjugated to the EPO via a linker molecule.

6. The HAS-EPO of any of claims 3 to 5, wherein HAS is conjugated to the EPO via a carbohydrate moiety which is part of the carbohydrate side chains and which is preferably oxidized.

7. The HAS-EPO of claim 6, wherein HAS is conjugated to a galactose or sialic acid residue of the carbohydrate side chains.

8. The HAS-EPO of any of claims 1 to 7, wherein the S atom in the thioether is derived from a naturally-occurring cysteine or from an added cysteine.

9. The HAS-EPO of claim 8, wherein the EPO has the amino acid sequence of human EPO and the naturally occurring cysteines are cysteine 29 and/ or 33.

10. The HAS-EPO of claim 9, wherein HAS is conjugated to cysteine 29 and cysteine 33 is replaced by another amino acid.

11. The HAS-EPO of claim 9, wherein HAS is conjugated to cysteine 33 and cysteine 29 is replaced by another amino acid.

12. The HAS-EPO of any of claims 8 to 11, wherein the added cysteine has been added by replacing a naturally occuring amino acid by a cysteine.

13. The HAS-EPO of claim 12, wherein the EPO is human EPO and the replaced amino acid residue is serine 126.

14. The HAS-EPO of any of claims 1 to 13, comprising 1-12, preferably 1-6 or 1-3, most preferred 1-4 HAS molecules per EPO molecule.

15. The HAS-EPO of any of claims 1 to 14, wherein the HAS is selected from the group consisting of hydroxyethylstarch, hydroxypropylstarch and hydroxybutylstarch.

16. The HAS-EPO of claim 15, wherein the HAS is hydroxyethylstarch (HES).

17. The HAS-EPO of claim 16, wherein the HES has a molecular weight of 1 to 300 kDa, preferably 5 to 100 kDa.

18. The HAS-EPO of any of claims 16 or 17, wherein the HES exhibits a molar degree of substitution of 0.1 to 0.8 and a ratio between C₂:C₆-substitution in the range of 2-20, with respect to the hydroxyethylgroups.

19. A method for the production of a hydroxyalkylstarch (HAS)-erythropoietin (EPO)-conjugate (HAS-EPO), comprising the steps of:
a) providing EPO being capable of reacting with modified HAS,
b) providing modified HAS being capable of reacting with the EPO of step a), and
c) reacting the EPO of step a) with the HAS of step b), whereby an HAS-EPO is produced comprising one or more HAS molecules, wherein each HAS is conjugated to the EPO via
i) a carbohydrate moiety; or
ii) a thioether.

20. The method of claim 19, wherein the EPO has the amino acid sequence of human EPO.

21. The method of any of claims 19 or 20, wherein the EPO is recombinantly produced.

22. The method of any of claims 19 to 21, wherein the EPO comprises one or more carbohydrate side chains attached to the EPO via N- and/ or O-linked glycosylation.

23. The method of claim 22, wherein the carbohydrate side chains have been attached to the EPO during production in mammalian, especially human, insect or yeast cells.

24. The method of any of claims 22 or 23, wherein the HAS is conjugated to the EPO via a carbohydrate moiety which is part of the carbohydrate side chains.

25. The method of claim 24, wherein in step a) the EPO is modified by oxidizing at least one carbohydrate moiety, preferably at least one terminal saccharide unit, more preferably galactose, of the one or more carbohydrate side chains of the EPO.

26. The method of claim 25, wherein the terminal saccharide unit is oxidized after partial or complete (enzymatic and/ or chemical) removal of the terminal sialic acid.

27. The method of claims 25 or 26, wherein in step c) the modified HAS is conjugated to the oxidized terminal saccharide unit.

28. The method of any of claims 19 to 27, wherein the EPO comprises at least one free SH-group.

29. The method of claim 28, wherein the free SH-group is part of a naturally-occurring cysteine or of an added cysteine.

30. The method of claim 29, wherein the EPO has the amino acid sequence of human EPO and the naturally occurring cysteines are cysteine 29 and/ or 33.

31. The method of claim 30, wherein cysteine 33 is replaced by another amino acid and in step c) the modified HAS is conjugated to cysteine 29.

32. The method of claim 30, wherein cysteine 29 is replaced by another amino acid and in step c) the modified HAS is conjugated to cysteine 33.

33. The method of any of claims 29 to 32, wherein the added cysteine has been added by replacing a naturally occuring amino acid by a cysteine.

34. The method of claim 33, wherein the EPO is human EPO and the replaced amino acid residue is serine 126.

35. The method of any of claims 33 or 34, wherein in step c) the modified HAS is conjugated to the added cysteine.

36. The method of any of claims 19 to 35, wherein the HAS is modified such that it comprises a free hydrazide, hydroxylamine, thiol or semicarbazide function if the HAS is conjugated to the oxidized carbohydrate moieties or a free maleimide, disulfide or halogen acetamide function if the HAS is to be conjugated to the SH-group.

37. The method of any of claims 19 to 36, wherein step c) is performed in a reaction medium comprising at least 10 % per weight H₂O.

38. The method of any of claims 19 to 37, wherein the HAS is conjugated to the EPO via a linker molecule.

39. The method of any of claims 19 to 38, wherein the HAS is, hydroxyethylstarch, hydroxypropylstarch or hydroxybutylstarch, preferably hydroxyethylstarch (HES).

40. The method of claim 39, wherein the HES has the properties as defined in any of claims 17 or 18.

41. A HAS-EPO, obtainable by the method of any of claims 19 to 40.

42. The HAS-EPO of claim 41, having the features as defined in any of claims 1 to 18.

43. A HAS-EPO according to any of claims 1 to 18, 41 or 42 for use in a method for treatment of the human or animal body.

44. A pharmaceutical composition comprising the HAS-EPO according to any of claims 1 to 18, 41 or 42.

45. The pharmaceutical composition of claim 44, further comprising at least one pharmaceutically acceptable carrier.

46. Use of a HAS-EPO according to any of claims 1 to 18, 41 or 42 for the preparation of a medicament for the treatment of anemic disorders or hematopoietic dysfunction disorders.

47. A hydroxyalkylstarch (HAS)-polypeptide-conjugate (HAS-polypeptide), comprising one or more HAS molecules, wherein each HAS is conjugated to the polypeptide via
c) a carbohydrate moiety; or
d) a thioether.

48. The HAS-polypeptide of claim 47, wherein the polypeptide is of human origin.

49. The HAS-polypeptide of any of claims 47 or 48, wherein the polypeptide is selected from the group comprising erythropoietin, interleukins, especially interleukin-2, IFN-ß, IFN-alpha, CSF, interleukin 6 and therapeutic antibodies.

50. The HAS-polypeptide of any of claims 47 to 49, wherein the polypeptide comprises one or more carbohydrate side chains attached to the polypeptide via N- and/ or O-linked glycosylation.

51. The HAS-polypeptide of claim 50, wherein the carbohydrate side chains have been attached to the polypeptide during production in mammalian, especially human, insect or yeast cells.

52. The HAS-polypeptide of any of claims 47 to 51, wherein the HAS is conjugated to the polypeptide via a linker molecule.

53. The HAS-polypeptide of any of claims 49 to 52, wherein the HAS is conjugated to the polypeptide via a carbohydrate moiety which is part of the carbohydrate side chains and which is preferably oxidized.

54. The HAS-polypeptide of claim 53, wherein the HAS is conjugated to a galactose residue of the carbohydrate side chains.

55. The HAS-polypeptide of any of claims 47 to 54, wherein the S atom in the thioether is derived from a naturally-occurring cysteine or from an added cysteine.

56. The HAS-polypeptide of claim 55, wherein the added cysteine has been added by replacing a naturally occuring amino acid by a cysteine.

57. The HAS-polypeptide of any of claims 47 to 56, comprising 1-12, preferably 1-6 or 1-3, most preferred 1-4 HAS molecules per polypeptide molecule.

58. The HAS-polxpeptide of any of claims 47 to 57, wherein the HAS is selected from the group consisting of hydroxyethylstarch, hydroxypropylstarch and hydroxybutylstarch.

59. The HAS-polypeptide of claim 58, wherein the HAS is hydroxyethylstarch (HES).

60. The HAS-polypeptide of claim 59, wherein the HES has a molecular weight of 1 to 300 kDa, preferably 5 to 100 kDa.

61. The HAS-polypeptide of any of claims 59 or 60, wherein the HES exhibits a molar degree of substitution of 0.1 to 0.8 and a ratio between C₂:C₆-substitution in the range of 2-20, with respect to the hydroxyethylgroups.

62. A method for the production of a hydroxyalkylstarch (HAS)-polypeptide-conjugate (HAS-polypeptide), comprising the steps of:
d) providing a polypeptide being capable of reacting with modified HAS,
e) providing modified HAS being capable of reacting with the polypeptide of step a), and
f) reacting the polypeptide of step a) with the HAS of step b), whereby HAS-polypeptide is produced comprising one or more HAS molecules, wherein each HAS is conjugated to the polypeptide via
i) a carbohydrate moiety; or
ii) a thioether.

63. The method of claim 62, wherein the polypeptide is of human origin.

64. The method of any of claims 62 or 63, wherein the polypeptide is selected from the group comprising erythropoietin, interleukins, especially interleukin-2, IFN-β, IFN-alpha, CSF, interleukin 6 and therapeutic antibodies

65. The method of any of claims 62 to 64, wherein the polypeptide is recombinantly produced.

66. The method of any of claims 62 to 65, wherein the polypeptide comprises one or more carbohydrate side chains attached to the polypeptide via N-and/ or O-linked glycosylation.

67. The method of claim 66, wherein the carbohydrate side chains have been attached to the polypeptide during production in mammalian, especially human, insect or yeast cells.

68. The method of any of claims 66 or 67, wherein the HAS is conjugated to the polypeptide via a carbohydrate moiety which is part of the carbohydrate side chains.

69. The method of claim 68, wherein in step a) the polypeptide is modified by oxidizing at least one carbohydrate moiety, preferably at least one terminal saccharide unit, more preferably galactose, of the one or more carbohydrate side chains of the polypeptide.

70. The method of claim 69, wherein the terminal saccharide unit is oxidized after partial or complete (enzymatic and/ or chemical) removal of the terminal sialic acid.

71. The method of claims 69 or 70, wherein in step c) the modified HAS is conjugated to the oxidized terminal saccharide unit.

72. The method of any of claims 62 to 71, wherein the polypeptide comprises at least one free SH-group.

73. The method of claim 72, wherein the free SH-group is part of a naturally-occurring cysteine or of an added cysteine.

74. The method of any of claims 62 to 73, wherein the added cysteine has been added by replacing a naturally occuring amino acid by a cysteine.

75. The method of any of claims 73 or 74, wherein in step c) the modified HAS is conjugated to the added cysteine.

76. The method of any of claims 62 to 75, wherein the HAS is modified such that it comprises a free hydrazide, hydroxylamine, thiol or semicarbazide function if the HAS is conjugated to the oxidized carbohydrate moieties or a free maleimide, disulfide or halogen acetamide function if the HAS is to be conjugated to the SH-group.

77. The method of any of claims 62 to 76, wherein step c) is performed in a reaction medium comprising at least 10 % per weight H₂O.

78. The method of any of claims 62 to 78, wherein the HAS is conjugated to the polypeptide via a linker molecule.

79. The method of any of claims 62 to 78, wherein the HAS is hydroxyethylstarch, hydroxypropylstarch or hydroxybutylstarch, preferably hydroxyethylstarch (HES).

80. The method of claim 79, wherein the HAS has the properties as defined in any of claims 60 or 61.

81. A HAS-polypeptide, obtainable by the method of any of claims 62 to 80.

82. The HAS-polypeptide of claim 41, having the features as defined in any of claims 47 to 61.

83. A HAS-polypeptide according to any of claims 47 to 61, 81 or 82 for use in a method for treatment of the human or animal body.

84. A pharmaceutical composition comprising the HAS-polypeptide according to any of claims 47 to 61, 81 or 82.

85. The pharmaceutical composition of claim 84, further comprising at least one pharmaceutically acceptable carrier.
